# EUROPEAN PATENT APPLICATION

(11) **EP 3 292 905 A1**
(43) Date of publication of application: **14.03.2018**
(21) Application number: 17193355.9
(22) Date of filing: 25.01.2013
(51) Int. Cl.: B01D 69/10, B01D 69/06, B01D 71/36, B01D 69/02, D04H 1/728

(54) **ELECTROSPUN POROUS MEDIA**

(30) Priority: 27.01.2012 US 201261591555 P; 01.03.2012 US 201261605510 P; 04.04.2012 US 201261620179 P; 04.10.2012 US 201261709450 P
(62) Divisional of application: 13741191.4
(71) Applicant: Zeus Industrial Products, Inc., Orangeburg, South Carolina 29118 (US)
(72) Inventor: BALLARD, Robert L., Orangeburg, SC South Carolina 29118 (US); ANNEAUX, Bruce L., Lexington, SC South Carolina 29073 (US); MANASCO, Joshua L., West Columbia, SC South Carolina 29172 (US); GARNER, David P., Lexington, SC South Carolina 29172 (US); HAO, Ping, Waxhaw, NC North Carolina 28173 (US)
(74) Representative: Hofstetter, Schurack & Partner

(57) **Abstract**

Espun material may function as a filtration medium or be put to other uses. The espun material may comprise espun poly(tetrafluoroethylene) (espun PTFE). One or more layers of the espun material may be included. The properties of the espun material can be tailored. For example, a gradient fabric may include espun PTFE. The gradient fabric may include two or more layers of espun PTFE.

## Description

### CROSS-REFERENCE TO RELATED APPLICATION

This application claims the benefit of each of U.S. Provisional Application No. 61,591,555, filed January 27, 2012; U.S. Provisional Application No. 61,605,510, filed March 1, 2012; U.S. Provisional Application No. 61,620,179, filed April 4, 2012; and U.S. Provisional Application No. 61,709,450, filed October 4, 2012.

### INCORPORATION BY REFERENCE

The entire contents of each of U.S. Provisional Application No. 61,591,555, filed January 27, 2012; U.S. Provisional Application No. 61,605,510, filed March 1, 2012; U.S. Provisional Application No. 61,620,179, filed April 4, 2012; and U.S. Provisional Application No. 61,709,450, filed October 4, 2012, is incorporated herein by reference.

### FIELD OF THE INVENTION

The present invention is generally related to porous media as well as to methods of making and using such porous media.

### BACKGROUND

Porous media may be used, for example and not for the purpose of limiting the scope of the present invention, as a filter or filtration medium for removing particulates from a gas or liquid stream. The choice of which type of filter (and thus, filtration medium) to use is dictated by the size and characteristics of the particles to be removed, the conditions of removal, and/or the gas or liquid source from which the particles are to be removed.

The flow of gases or liquids through a filter medium typically produces a pressure differential or pressure drop. Preferably, the pressure drop associated with a given filtration process should be as small as possible to minimize the energy required to filter the gas or liquid. However, over time, the pressure drop tends to increase due to the accumulation of particulate matter in the filter medium. When the pressure drop becomes excessive, the filter must either be replaced or cleaned, which may be a time-consuming and/or expensive process.

Filter media generally may be characterized as being either depth filtration media or surface filtration media. For surface filtration, the particles are collected and accumulate on the surface of the filter medium. Most surface filters are made from microporous membranes. Surface filtration media usually provides the most effective filtration efficiency. The structure of the membrane with millions of pores is such that submicron particles are captured on its surface. Generally, the backing substrate of surface filtration membrane may merely be a support that plays a minimal part in the filtration process, since the particulate is collected on the surface of the membrane. That is, there is typically no penetration of the particulate into or beyond the filter media. As compared to depth filtration, favorable characteristics of surface filtration media relate to smaller housings, which means fewer cartridges/dust bags are required to achieve the same level of filtration efficiency. Relative to depth filtration, surface filtration is more suitable for fine particulate capture, such as for particulates having a maximal dimension of less than 10 µm. Although more expensive initially than depth filtration media, surface filtration media typically has a less frequent replacement cycle and lower disposal costs.

For depth filtration, the particles tend to penetrate the filter medium and become captured throughout the depth of the media. Depth filtration media has a broad pore size distribution and relies on adsorption-retention for a portion of its dirt holding capacity. Some depth filters have a gradient pore structure to maximize mechanical retention. Most depth filters are made from coarse fibers such as meltblown nonwovens that are much more open than surface filters. The interstices between the fibers within the structure of the depth filtration media are often considerably larger than the particles to be collected. The dust particles penetrate the surface of the media to "close off' the open pores, blind the media, and increase the pressure drop. As a result, atmospheric emissions can occur over time as individual dust particles penetrate into and beyond the filter media. Conventional depth filtration media are rarely able to collect fine particulate efficiently. As compared to surface filtration media, depth filtration media are more suitable for removing a broader range of particles having a maximal dimension of greater than 10 µm. As compared to surface filtration media, the depth filtration media is more economical initially, but has a shorter filter life, higher frequency replacement, and a higher disposal cost.

Examples of depth filtration media include spunbond and meltblown fabrics. These materials may be made from a variety of materials that are melt processable. In use, meltblown filter media typically demonstrate high filtration efficiency with a relatively low pressure drop.

An example of a surface filtration medium is an expanded poly(tetrafluoroethylene) (ePTFE) material. ePTFE membranes are advantageous in that they are typically capable of performing well even in harsh environments and at high temperatures. ePTFE membranes are typically highly efficient in filtration, but can often be associated with a high pressure drop.

In general, poly(tetrafluoroethylene) (PTFE) is a thermoplastic that offers exceptional resistance to high temperatures and corrosive environments, yet is inert, nontoxic, and is often used in body implants. PTFE is impossible to process by conventional molten polymer techniques (other than matrix molding) but can be extruded as a paste and then drawn into various forms to produce fibers, ribbons, membranes, or tubes. ePTFE is made in this fashion. An alternative means for processing PTFE is to provide a dispersion of PTFE that can be used to form coatings or alternatively combined with fiber forming polymers and electrostatically spun ("electrospun" or "espun") to produce nonwoven fabrics, coverings, bats, or composites based on nanofibers. These forms of PTFE are commonly sintered or partially sintered at high temperatures to develop desirable mechanical properties. Processes of electrostatic spinning have been described previously, as provided for example in U.S. Patent Nos. 2,158,416; 4,432,916; 4,287,139; 4,143,196; 4,043,331; 4,689,186 and 6,641,773, each of which is incorporated herein by reference in its entirety. Further, U.S. Patent Nos. 4,323,525, 4,044,404 and 4,043,331, which are incorporated herein by reference in their entireties, provide information related to processing and electrostatic spinning of PTFE from an aqueous or other dispersion into a fabric that is subsequently sintered to achieve a final stable form. For both ePTFE and electrospun PTFE, a porous structure is created with high surface area. Advantageously, higher viscosity dispersions have been recently used to achieve more uniform materials, as demonstrated in U.S. Patent No. 8,178,030 to Anneaux et al., which is incorporated herein by reference.

It would be beneficial to provide alternative porous media such as, but not limited to, filtration media for use in various applications.

### SUMMARY

One aspect of this disclosure is the provision of an espun material that is porous and, in at least some examples, is configured for functioning as a filtration medium. Preferably (e.g., optionally), the espun material comprises espun poly(tetrafluoroethylene) (espun PTFE). In some embodiments, one or more additional layers may be included.

In certain aspects of this disclosure that relate to filtration media, a filter may comprise a support structure and an espun mat comprising, or consisting essentially of, espun PTFE supported on the support structure. In certain aspects of this disclosure, a filtering system comprises a filter assembly removably mounted in a forced fluid path (e.g., gas or liquid), wherein the filter assembly includes a support structure and an espun PTFE mat supported thereon.

In some embodiments, a filtration medium may comprise an espun PTFE mat capable of meeting HEPA standard IEST-RP-CC001.3, wherein: the espun PTFE mat comprises nonwoven fibers having an average fiber diameter between about 250 nm and about 1500 nm, and the average thickness of the espun PTFE mat is about 200 µm or less; the espun PTFE mat comprises nonwoven fibers having an average fiber diameter between about 463 nm and about 1500 nm, and the average thickness of the espun PTFE mat is about 200 µm or less; or the espun PTFE mat comprises nonwoven fibers having an average fiber diameter between about 1500 nm and three times the average particle size of poly(tetrafluoroethylene) particulates in a dispersion electrospun to form the mat, and the average thickness of the espun PTFE mat is about 200 µm or less. In some embodiments, the average fiber diameter may be less than about 500 nm or less than about 400 nm. Other fiber diameters are within the scope of this disclosure.

The average thickness of the espun PTFE mat can vary and in certain embodiment, may be about 100 µm or less. The pressure drop over the espun PTFE mat is advantageously low. For example, in some embodiments, the pressure drop may be about 40 mm H₂O or less or about 30 mm H₂O or less. The density of the espun PTFE mat can vary. For example and in accordance with some embodiments, the density of the espun PTFE mat may be about 30 grams per square meter per mil thickness or less, about 25 grams per square meter per mil thickness or less, about 20 grams per square meter per mil thickness or less, or about 10 grams per square meter per mil thickness or less.

Another aspect of this disclosure is the provision of a method for preparing a porous espun material, which may optionally be used as a filtration medium, wherein the method comprises: providing a dispersion comprising a fluorinated polymer in particulate form with a given average particle size, a fiberizing polymer, a solvent or other suitable dispersion medium, and an optional conductive species, such that the dispersion has a given conductivity; and electro spinning the dispersion to provide a polymeric mat capable of meeting HEPA standard IEST-RP-CC001.3. The fluorinated polymer can vary and may be, for example, poly(tetrafluoroethylene) or a polymer selected from the group consisting of fluorinated ethylene propylene (FEP), polyvinylidene fluoride (PVDF), perfluoroalkoxy (PFA), a copolymer of tetrafluoroethylene, hexafluoropropylene and vinylidene fluoride (THV), poly(ethylene-co-tetrafluoroethylene) (ETFE), ethylene chlorotrifluoroethylene (ECTFE), polychlorotrifluoroethylene (PCTFE), and copolymers, blends, and derivatives thereof.

The conductive species may be any reagent that dissociates into ions in water and which does not disrupt the structure of the dispersion. The conductive species may be, for example, a water-soluble salt, e.g., an ammonium salt. The given average particle size of the fluorinated polymer in the dispersion can vary and may, in some embodiments, be about 230 nm or less; about 160 nm or less; about 130 nm or less; or about 80 nm or less. In some embodiments, the electrospinning step is performed for a period of time such that the polymeric mat has an average thickness of about 200 µm or less (e.g., about 100 µm or less).

The polymeric mats of this disclosure may be put to a variety of uses. Depending upon the end uses and for example, the polymeric mats may be supported and/or strength may be imparted to the polymeric mats in any suitable manner. For example, in an embodiment in which a polymeric mat of this disclosure is part of a filter, the filter may include a support structure, and the support structure may comprise a frame that extends at least partially around, or more specifically extends all the way around, and encloses the periphery of the polymeric mat. The support structure may further include cross-members that are connected to the frame and extend across the opposite faces of the polymeric mat, so that the polymeric mat is positioned between opposite groups of the cross-members. In addition and/or alternatively, the support structure may include or be in the form of one or more layers of material that may be superposed with one or more layers of the polymeric mats of this disclosure. The layers may be connected to one another in any suitable manner, such as through laminating or mechanical fastening, and the one or more layers that are in addition to the electrospun polymer layer(s) may provide any suitable functions in addition to, or as an alternative to, the support function.

For each polymeric mat of this disclosure, strength may be imparted to the polymeric mat by bonding together portions of the fibers of the polymeric mat, typically while maintaining, or at least substantially maintaining, the porosity of the mat. For example, this bonding may be provided by way of sintering or a process that includes sintering. For example, the bonding may comprise some melting, although any such melting may be controlled or otherwise minimized in an effort to control (e.g., maintain) the porosity of the polymeric mat. For example and in accordance with one aspect of this disclosure, a filter may comprise an electrospun polymer mat having fibers that have been sintered and/or thermally fused together.

In accordance with certain embodiments of the present disclosure, filtration media comprising, or consisting essentially of, electrospun PTFE in the form of a gradient fabric are provided. In some embodiments, a filter comprises a support structure and an electrospun PTFE gradient fabric filtration medium supported thereon. In certain embodiments, a filtering system comprising a filter with a support structure and an electrospun PTFE gradient fabric filtration medium supported thereon is provided, wherein the filter is removably mounted in a forced fluid path (e.g., gas or liquid).

Electrospun PTFE can, in certain embodiments, provide benefits typically associated with both two-dimensional and three-dimensional filtration performances. As a result, in some aspects of this disclosure, a material is provided that is capable of bridging the range of filtration capabilities of the two types of media and broadening the spectrum of filtration applications. In certain embodiments, electrospun PTFE has depth filtration (three-dimensional) characteristics in that it reduces flow resistance and lowers the pressure drop, yet offers surface membrane (two-dimensional) characteristics for higher filtration efficiency and particulate release. In some aspects of this disclosure, electrospun PTFE offers yet another potential advantage: the ability to control the effective pore size of a filtration medium not only by thickness, but also by density.

One aspect of this disclosure is the provision of a gradient fabric consisting of two or more layers of espun PTFE fibers, wherein the two or more layers comprise at least two layers having different densities, such that the cross-section of the fabric exhibits one or more density gradients, wherein air flow through the cross-section of the fabric results in a filtration efficiency that is enhanced relative to the filtration efficiency through any of the layers independently and a pressure drop that is within the range of pressure drops exhibited by any of the layers independently.

In specific embodiments, the gradient fabric can consist of three or more layers. The two or more layers (e.g., three or more layers) can be, in some embodiments, continuous along the length and width of the fabric. The thickness of the layers can vary; for example, the thickness of each layer can be between about 0.5 µ and about 1000 µ.

In some embodiments, the density gradient comprises layers having increasing densities across the thickness of the cross-section, such that the air flow passes from a least dense layer to a most dense layer or from a less dense layer to a more dense layer. In other embodiments, the density gradient comprises layers having decreasing densities across the thickness of the cross-section, such that the air flow passes from a most dense layer to a least dense layer, or from a more dense layer to a less dense layer. In still other embodiments, the density gradient comprises layers having increasing densities and decreasing densities across the thickness of the cross-section. In certain embodiments, the layer with the highest density is on the interior of the cross-section, such that air flow passes first through one or more layers with lower densities, then through the layer with the highest density, and then through one or more layers with lower densities. The density gradient may, in some embodiments, comprise a substantially uniform gradient in density across the cross-section of the fabric. In some embodiments, the gradient fabric can further comprise one or more additional components, e.g., a substrate to which the fabric is attached.

As additional examples, the electrospun polymer mats of this disclosure may also be used as, or be incorporated into, separation membranes, engineered fabrics, and tissue scaffolding, or they may be used in any other suitable manner. For these and other purposes, a mat comprising, consisting of, or consisting essentially of electrospun poly(tetrafluoroethylene) may have an average fiber diameter of less than about 1003 nm, and a density of greater than about 8.1 grams per square meter per mil thickness or less.

As another specific example, the espun PTFE mats of this disclosure may be configured to be, or may be part of, a breathable material that both prevents (e.g., substantially prevents) penetration of water and/or other liquids, while allowing the release of moisture vapor and air to provide thermal comfort. In one specific example, a nonwoven fabric comprises or consists essentially of electrospun poly(tetrafluoroethylene), wherein the nonwoven fabric is configured so that it is capable of both passing the blood penetration test of ASTM F1670 and providing air permeability of at least about 2.3 cfm, or at least about 3 cfm, wherein the air permeability may be in a range extending from one of those values to less than about 9.0 cfm, or less than about 8.5 cfm, or in any other suitable range. As more specific examples, an espun PTFE nonwoven fabric that passes the blood penetration test of ASTM F1670 may have: a thickness of about 20 µ, an air permeability of about 8.14 cfm, and a hydro head of about 442 cm; a thickness of about 40 µ, an air permeability of about 6.24 cfm, and a hydro head of about 474 cm; a thickness of about 60 µ, an air permeability of about 4.43 cfm, and a hydro head of about 533 cm; a thickness of about 22 µ, an air permeability of about 9.96 cfm, and a hydro head of about 395 cm; and/or a thickness of about 68 µ, an air permeability of about 2.98 cfm, and a hydro head of about 664 cm.

A variety of espun PTFE nonwoven mats and uses thereof are within the scope of this disclosure, so that the above-presented features and values, like other features and values provided throughout this disclosure, may be utilized in various combinations and subcombinations, such as for providing a variety of quantifications (e.g., values, ranges of values, and/or the like) for defining aspects of the present invention.

Further aspects of the invention relate to the following points:
Point 1. A filter comprising:
   a support structure; and
   a mat comprising electro spun poly(tetrafluoroethylene), the mat being supported by the support structure.
Point 2. The filter according to point 1, wherein the filter is a fluid filter.
Point 3. The filter according to point 1 in combination with a forced fluid path, wherein the filter is removably mounted in the forced fluid path.
Point 4. A filtration medium comprising:
   a mat comprising electrospun poly(tetrafluoroethylene), the mat being configured so that the mat is capable of meeting HEPA standard IEST-RP-CC001.3, the electrospun poly(tetrafluoroethylene) comprising nonwoven fibers having an average fiber diameter between about 250 nm and about 1500 nm, and an average thickness of the mat being about 200 µm or less.
Point 5. The filtration medium of point 4, wherein the mat comprises a nonwoven fabric, and the nonwoven fabric comprises the electrospun poly(tetrafluoroethylene).
Point 6. The filtration medium of point 4, wherein the average fiber diameter is about 500 nm or less.
Point 7. The filtration medium of point 4, wherein the average fiber diameter is about 400 nm or less.
Point 8. The filtration medium of point 4, wherein the mat is a mat of the electrospun poly(tetrafluoroethylene), and an average thickness of the electrospun poly(tetrafluoroethylene) mat is about 100 µm or less.
Point 9. The filtration medium of point 8, wherein the electrospun poly(tetrafluoroethylene) mat is configured for providing a pressure drop of about 40 mm H₂O or less when tested under HEPA MIL-STD 282 using 0.3 µm particles, 5.3 cm/s velocity, and a 100 cm2 area of the electrospun poly(tetrafluoroethylene) mat in a flat configuration.
Point 10. The filtration medium of point 8, wherein the electrospun poly(tetrafluoroethylene) mat is configured for providing a pressure drop of about 4 mm H₂O or less when tested under HEPA MIL-STD 282 using 0.3 µm particles, 5.3 cm/s velocity, and a 100 cm2 area of the electrospun poly(tetrafluoroethylene) mat in a flat configuration.
Point 11. The filtration medium of point 8, wherein the density of the electrospun poly(tetrafluoroethylene) mat is about 30 grams per square meter per mil thickness or less.
Point 12. The filtration medium of point 11, wherein the density of the electrospun poly(tetrafluoroethylene) mat is about 25 grams per square meter per mil thickness or less.
Point 13. The filtration medium of point 12, wherein the density of the electrospun poly(tetrafluoroethylene) mat is about 20 grams per square meter per mil thickness or less.
Point 14. The filtration medium of point 13, wherein the density of the electrospun poly(tetrafluoroethylene) mat is about 10 grams per square meter per mil thickness or less.
Point 15. A method for preparing a filtration medium, comprising:
   having a dispersion comprising
      a fluorinated polymer in particulate form with a given average particle size;
      a fiberizing polymer;
      a dispersion medium; and
   an optional conductive species; such that the dispersion has a given conductivity; and
   electrospinning said dispersion to provide a polymeric mat capable of meeting HEPA standard IEST-RP-CC001.3.
Point 16. The method of point 15, wherein the fluorinated polymer is poly(tetrafluoroethylene).
Point 17. The method of point 15, wherein the fluorinated polymer is selected from the group consisting of fluorinated ethylene propylene, polyvinylidene fluoride, perfluoroalkoxy, a copolymer of tetrafluoroethylene, hexafluoropropylene and vinylidene fluoride (THV), poly(ethylene-co-tetrafluoroethylene), ethylene chlorotrifluoroethylene, polychlorotrifluoroethylene, and copolymers, blends, and derivatives thereof.
Point 18. The method of point 15, wherein the conductive species is a water-soluble salt.
Point 19. The method of point 15, wherein the conductive species is ammonium hydroxide.
Point 20. The method of point 15, wherein the given average particle size of the fluorinated polymer is about 230 nm or less.
Point 21. The method of point 20, wherein the given average particle size of the fluorinated polymer is about 160 nm or less.
Point 22. The method of point 21, wherein the given average particle size of the fluorinated polymer is about 130 nm or less.
Point 23. The method of point 22, wherein the given average particle size of the fluorinated polymer is about 80 nm or less.
Point 24. The method of point 15, wherein the electrospinning step is performed for a period of time such that the polymeric mat has an average thickness of about 200 µm or less.
Point 25. The method of point 15, wherein the electrospinning step is performed for a period of time such that the polymeric mat has an average thickness of about 100 µm or less.
Point 26. A filtration medium comprising:
   a nonwoven, electrospun poly(tetrafluoroethylene) mat capable of functioning as an ULPA filter so as to remove from air at least 99.999% of airborne particles with particle sizes of less than 0.3 µ, wherein
   the electrospun poly(tetrafluoroethylene) mat comprises nonwoven fibers having an average fiber diameter between about 250 nm and about 1500 nm, and
   an average thickness of the electrospun poly(tetrafluoroethylene) mat is about 200 µm or less.
Point 27. The filtration medium of point 26, wherein the average fiber diameter is about 500 nm or less.
Point 28. The filtration medium of point 26, wherein the average fiber diameter is about 400 nm or less.
Point 29. The filtration medium of point 26, wherein the average thickness of the electrospun poly(tetrafluoroethylene) mat is about 100 µm or less.
Point 30. The filtration medium of point 26, wherein the pressure drop over the electrospun poly(tetrafluoroethylene) mat is about 40 mm H₂O or less when tested under HEPA MIL-STD 282 using 0.3 µm particles, 5.3 cm/s velocity, and a 100 cm2 area of the electrospun poly(tetrafluoroethylene) mat in a flat configuration..
Point 31. The filtration medium of point 26, wherein the pressure drop over the electrospun poly(tetrafluoroethylene) mat is about 30 mm H₂O or less when tested under HEPA MIL-STD 282 using 0.3 µm particles, 5.3 cm/s velocity, and a 100 cm2 area of the electrospun poly(tetrafluoroethylene) mat in a flat configuration.
Point 32. The filtration medium of point 26, wherein the density of the electrospun poly(tetrafluoroethylene) mat is about 30 grams per square meter per mil thickness or less.
Point 33. The filtration medium of point 32, wherein the density of the electrospun poly(tetrafluoroethylene) mat is about 25 grams per square meter per mil thickness or less.
Point 34. The filtration medium of point 33, wherein the density of the electrospun poly(tetrafluoroethylene) mat is about 20 grams per square meter per mil thickness or less.
Point 35. A method for preparing a filtration medium, comprising:
   having a dispersion comprising:
      a fluorinated polymer in particulate form with a given average particle size;
      a fiberizing polymer;
      a dispersion medium; and
   an optional conductive species; such that the dispersion has a given conductivity; and
   electrospinning said dispersion to provide a polymeric mat capable of functioning as an ULPA filter so as to remove from air at least 99.999% of airborne particles with a particle size of less than 0.3 microns.
Point 36. The method of point 35, wherein the fluorinated polymer is poly(tetrafluoroethylene).
Point 37. The method of point 35, wherein the fluorinated polymer is selected from the group consisting of fluorinated ethylene propylene, polyvinylidene fluoride, perfluoroalkoxy, a copolymer of tetrafluoroethylene, hexafluoropropylene and vinylidene fluoride (THV), poly(ethylene-co-tetrafluoroethylene), ethylene chlorotrifluoroethylene, polychlorotrifluoroethylene, and copolymers, blends, and derivatives thereof.
Point 38. The method of point 35, wherein the conductive species is a water-soluble salt.
Point 39. The method of point 35, wherein the conductive species is ammonium hydroxide.
Point 40. The method of point 35, wherein the given average particle size of the fluorinated polymer is about 230 nm or less.
Point 41. The method of point 40, wherein the given average particle size of the fluorinated polymer is about 160 nm or less.
Point 42. The method of point 41, wherein the given average particle size of the fluorinated polymer is about 130 nm or less.
Point 43. The method of point 42, wherein the given average particle size of the fluorinated polymer is about 80 nm or less.
Point 44. The method of point 35, wherein the electrospinning step is performed for a period of time such that the polymeric mat has an average thickness of about 200 µm or less.
Point 45. The method of point 35, wherein the electrospinning step is performed for a period of time such that the polymeric mat has an average thickness of about 100 µm or less.
Point 46. A mat comprising electrospun poly(tetrafluoroethylene), the mat comprising:
   an average fiber diameter of less than about 1003 nm; and
   a density of greater than about 8.1 grams per square meter per mil thickness or less.
Point 47. The mat of point 46, wherein
   the average fiber diameter is less than about 852 nm; and
   the density is greater than about 9.6 grams per square meter per mil thickness.
Point 48. A separation membrane comprising the mat of point 46.
Point 49. A fabric comprising the mat of point 46.
Point 50. A tissue scaffold comprising the mat of point 46.
Point 51. The tissue scaffold according to point 50 in combination with living cells, wherein the living cells are growing at least on the tissue scaffold.
Point 52. The tissue scaffold according to point 50 in combination with living cells, wherein the living cells are growing at least in pores of the tissue scaffold.
Point 53. A method for preparing a mat, comprising:
   having a dispersion comprising
   a fluorinated polymer in particulate form with an average particle size of less than about 230 nm,
   a fiberizing polymer,
   a dispersion medium, and
   a conductivity of more than about 250 µS/cm;
   electrospinning the dispersion to provide the mat.
   Point 54. The method of point 53, wherein the fluorinated polymer is poly(tetrafluoroethylene).
Point 55. The method of point 53, wherein:
   the average particle size is about 160 or less, and
   the conductivity is about 300 µS/cm or more.
Point 56. A gradient fabric comprising:
   two or more layers of electrospun poly(tetrafluoroethylene) fibers, wherein the two or more layers comprise at least two layers having different densities, such that a cross-section of the fabric exhibits one or more density gradients.
Point 57. The gradient fabric of point 56, wherein air flow through a cross-section of the fabric results:
   in a filtration efficiency that is enhanced relative to filtration efficiency through any of the layers independently, and
   a pressure drop that is within the range of pressure drops exhibited by any of the layers independently.
Point 58. The gradient fabric of point 56, wherein the gradient fabric consists of three or more layers.
Point 59. The gradient fabric of point 56 wherein the layers are continuous along a length and width of the fabric.
Point 60. The gradient fabric of point 56 wherein a thickness of each layer is between about 0.5 µ and about 1000 µ.
Point 61. The gradient fabric of point 56 wherein the density gradient comprises layers having increasing densities across a thickness of the cross-section.
Point 62. The gradient fabric of point 56, wherein the density gradient comprises layers having increasing densities and decreasing densities across the thickness of the cross-section.
Point 63. The gradient fabric of point 56, wherein a layer with the highest density is on the interior of the cross-section.
Point 64. The gradient fabric of point 56, wherein the density gradient comprises a substantially uniform gradient in density across a cross-section of the fabric.
Point 65. A nonwoven fabric comprising:
   electrospun poly(tetrafluoroethylene), the nonwoven fabric being configured so that the nonwoven fabric is capable of both
   passing the blood penetration test of ASTM F1670, and
   providing air permeability of at least about 2.5 cfm.

The foregoing presents a simplified summary of some aspects of this disclosure in order to provide a basic understanding. The foregoing summary is not extensive and is not intended to identify key or critical elements of the invention or to delineate the scope of the invention. The purpose of the foregoing summary is to present some concepts of this disclosure in a simplified form as a prelude to the more detailed description that is presented later. For example, other aspects will become apparent from the following.

### BRIEF DESCRIPTION OF THE DRAWINGS

A full and enabling disclosure, including the best mode thereof, directed to one of ordinary skill in the art, is set forth more particularly in the remainder of the specification, which makes reference to the following figures.
Fig. 1 is a schematic illustration of an orifice (needle)-based electrospinning apparatus that may be used according to one aspect of this disclosure.
Fig. 2 is a scanning electron micrograph ("SEM") of a mat of electrospun PTFE fibers in accordance with a comparative example, wherein the fibers are 1003 ± 105 nm in diameter.
Fig. 3 is a SEM of a mat of electrospun PTFE fibers in accordance with a first exemplary embodiment of this disclosure, wherein the fibers are 852 ± 141 nm (e.g., about 850 nm) in diameter.
Fig. 4A is a low-resolution SEM micrograph of a mat of electrospun PTFE fibers in accordance with a second exemplary embodiment of this disclosure, wherein the fibers are 463 ± 113 nm in diameter.
Fig. 4B is high-resolution SEM micrograph of the mat of Fig. 4A.
Fig. 5 is a SEM of a mat of electrospun PTFE fibers in accordance with a third exemplary embodiment of this disclosure, wherein the fibers are 651 ± 111 nm (e.g., about 650 nm) in diameter.
Fig. 6 is a SEM of a mat of electrospun PTFE fibers in accordance with a fourth exemplary embodiment of this disclosure, wherein the fibers are 609 ± 86 nm (e.g., about 550 nm) in diameter.
Fig. 7 is a graph (e.g., bar chart) providing a comparison of mean pore size versus thickness of various espun PTFE nonwovens.
Fig. 8 is a graph (e.g., bar chart) providing a comparison of porosity (the Gurley unit) versus thickness of various espun PTFE nonwovens.
Fig. 9 is a schematic, side cross-sectional view of homogeneous espun PTFE upon a stainless steel foil sheet, in accordance with an embodiment of this disclosure.
Fig. 10 is a schematic, side cross-sectional view of gradient espun PTFE upon a stainless steel foil sheet, in accordance with an embodiment of this disclosure.
Fig. 11 is a schematic, front pictorial view of a filter assembly, wherein a rear pictorial view of the filter assembly may be substantially the same as the front pictorial view, in accordance with an embodiment of this disclosure.
Fig. 12 is a chart illustrating exemplary ranges of water resistance and air permeability characteristics of some embodiments of this disclosure.

### DETAILED DESCRIPTION

Embodiments of this disclosure will be described more fully hereinafter. This invention may, however, be embodied in many different forms and should not be construed as limited to the embodiments set forth herein; rather, these embodiments are provided so that this disclosure will be thorough and complete, and will fully convey the scope of the invention to those skilled in the art. In fact, it will be apparent to those skilled in the art that various modifications and variations can be made in the present disclosure without departing from the scope or spirit of the invention. For instance, features illustrated or described as part of one embodiment may be used in another embodiment to yield a still further embodiment. Thus, it is intended that the present disclosure covers such modifications and variations as come within the scope of the appended claims and their equivalents. As used in this specification and the claims, the singular forms "a," "an," and "the" include plural referents unless the context clearly dictates otherwise.

An aspect of this disclosure is related to nonwovens (which may also be referred to herein as "fabrics," "sheets," "membranes," "mats" and/or "filtration media") comprising, or consisting essentially of, electrospun (which may also be referred to herein as "espin," "espun" and/or "espinning") fibers. In certain embodiments, the nonwovens comprise electrospun poly(tetrafluoroethylene) (espun PTFE); however, other embodiments are within the scope of this disclosure. That is, whereas the polymer PTFE is often referred to in the Detailed Description section of this disclosure, other suitable polymers may alternatively or additionally be used, such as, but not limited to, other fluorinated polymers. Stated differently, although some areas of the Detailed Description section of this disclosure specifically refer to PTFE, other types of polymer may be used. For example and in accordance with some embodiments, any suitable polymer that is capable of being provided in dispersion form may be used in place of PTFE in the methods detailed herein. In some specific embodiments, the polymer comprises a fluorinated polymer. For example, polymers that may be electrospun according to the present disclosure include, but are not limited to, fluorinated ethylene propylene (FEP), polyvinylidene fluoride (PVDF), perfluoroalkoxy (PFA), a copolymer of tetrafluoroethylene, hexafluoropropylene and vinylidene fluoride (THV), poly(ethylene-co-tetrafluoroethylene) (ETFE), ethylene chlorotrifluoroethylene (ECTFE), PCTFE (polychlorotrifluoroethylene), polyether ether ketone (PEEK), and copolymers, blends, and derivatives thereof.

General information related to processes for processing and electrostatic spinning of PTFE from aqueous and other dispersions is provided, for example, in U.S. Patent Nos. 4,323,525 to Bornat and 4,044,404 to Martin et al., which are incorporated herein by reference in their entireties. In certain embodiments, the electrospinning process used according to this disclosure is based, at least in part, on the process described in U.S. Patent No. 8,178,030 to Anneaux et al., which is incorporated herein by reference in its entirety.

In one aspect, the present disclosure provides for control of the properties of electrospun materials by modification of certain process parameters. Nonwovens that may be produced based on the present disclosure can function, for example and not for the purpose of limiting the scope of the present invention, as filtration media and may provide a number of advantages over conventional filtration materials. For example and in accordance with some embodiments, the preparation of espun nonwovens for use as filtration media allows for some degree of control over both microscopic properties (e.g., fiber diameter and/or fiber packing) and macroscopic properties (e.g., pore size, uniformity, and filtration performance).

### Electrospinning Process

Electrospun mats can be prepared by drawing material by electrical charge from a dispersion (e.g., solution or suspension/dispersion). The fibers thus produced are typically collected in a random fashion to produce nonwoven materials.

In certain embodiments, electrospinning of PTFE may be based at least in part, on the process described in detail in U.S. Patent No. 8,178,030 to Anneaux et al. and U.S. Patent Appl. Publ. No. 2012/0114722 to Ballard et al., which are both incorporated herein by reference in their entireties.

Various types of polymers that can be provided in solution form can be electrospun. In certain embodiments, electrospun mats prepared via solution electrospinning can be incorporated into the composites of this disclosure. Exemplary polymers that can be electrospun from solution include, but are not limited to, nylons, polyurethanes (PU), polyesters, and copolymers, blends, and derivatives thereof.

In certain embodiments, one or more electrospun fibrous layers are provided by dispersion-based electrospinning. In dispersion-based electrospinning, most types of polymers that are capable of being provided in dispersion form can be electrospun. In some embodiments, the polymer comprises poly(tetrafluoroethylene) (PTFE). Although much of the discussion provided herein focuses on PTFE, it is noted that other polymers can be used in place of PTFE in the methods detailed herein. For example, in some embodiments, polyether ether ketone (PEEK) is electrospun to provide a fibrous mat. In some specific embodiments, the electrospun fibrous mat comprises a fluoropolymer. Exemplary polymers that can be electrospun according to the present disclosure include, but are not limited to, fluorinated ethylene propylene (FEP), polyvinylidene fluoride (PVDF), perfluoroalkoxy (PFA), a copolymer of tetrafluoroethylene, hexafluoropropylene and vinylidene fluoride (THV), poly(ethylene-co-tetrafluoroethylene) (ETFE), ethylene chlorotrifluoroethylene (ECTFE), PCTFE (polychlorotrifluoroethylene), and copolymers, blends, and derivatives thereof.

An example of a suitable method for the production of a nonwoven PTFE material is described in the following, in accordance with various embodiments of this disclosure. Generally described, a dispersion that is electrospun, which may be referred to as an espinning dispersion, may be prepared by modifying a precursor dispersion. In accordance with some embodiments of this disclosure, both the precursor dispersions and the espinning dispersions include PTFE particles; therefore, they may be referred to as PTFE precursor dispersions and PTFE espinning dispersions. The PTFE precursor and espinning dispersions are typically intimately mixed dispersions. Whereas the present disclosure includes examples in which PTFE espinning dispersions are created from PTFE precursor dispersions, this disclosure is not limited to such examples. For example and at least theoretically, a PTFE espinning dispersion may be created without first creating a PTFE precursor dispersion. For example, a PTFE espinning dispersion may be created by simultaneously supplying all of the desired additives to the dispersion medium (e.g., solvent).

The solids content of (i.e. and/or e.g., the content of the PTFE particles in) the PTFE precursor dispersion, and optionally also in the PTFE espinning dispersion, is preferably (e.g., optionally) between about 50% to about 80% by weight, and more preferably (e.g., optionally) between about 55% and about 65% by weight. PTFE precursor dispersions useful according to some aspects of the present disclosure are commercially available, or they may be prepared by combining PTFE with, for example, one or more solvents or other suitable dispersion mediums. One example of a commercially available PTFE precursor dispersion is Daikin D 210 PTFE, which comprises about 59-61 wt % PTFE solids (measured according to ASTM D 4441), 6.0-7.2% wt % surfactant, a pH at 25 °C of 8.5 to 10.5, a specific gravity of 1.50 to 1.53 and a Brookfield viscosity maximum of 35 cP. Other PTFE precursor dispersions having different characteristics may be used. Specific examples of suitable PTFE precursor dispersions are discussed in greater detail below, in accordance with exemplary embodiments of this disclosure.

The properties of the PTFE (e.g., molecular weight, polydispersity index, particle size, particle size distribution) can vary. In some embodiments, the average particle size of the PTFE particles in the PTFE precursor and espinning dispersions may be between about 0.05 µm and about 1 µm (for example, between about 0.1 µm and about 0.5 µm). In some embodiments, the average particle size of the PTFE in the PTFE precursor and espinning dispersions is less than about 0.5 µm, less than about 0.4 µm, less than about 0.3 µm, or less than about 0.2 µm. For example and in accordance with some embodiments, the average particle size of the PTFE in the PTFE precursor and espinning dispersions may be about 0.08 µm, about 0.13 µm, about 0.16 µm, about 0.23 µm, or about 0.25 µm. The dispersion medium may be any solvent or other dispersion medium that is suitable for creating a dispersion, including, but not limited to, an aqueous solution or an alcohol solution (e.g., methanol, ethanol, or isopropanol).

As alluded to above, PTFE espinning dispersions may be created from PTFE precursor dispersions, such as by adding one or more additives to the PTFE precursor dispersions. A variety of additives are within the scope of this disclosure, as discussed in greater detail below. For example, the PTFE espinning dispersions generally further comprises one or more fiberizing polymers, and the fiberizing polymers may be added to the PTFE precursor dispersions.

A fiberizing polymer is any polymer sufficient to facilitate the formation of a nonwoven web and which can preferably (e.g., optionally) be removed following the electrospinning process to provide a PTFE-based electrospun material. The fiberizing polymer is typically selected such that it has a high solubility in the dispersion medium (e.g., solvent) of the dispersion. For example, where the solvent (e.g., dispersion medium) is water, any suitable water-soluble polymer can be used as the fiberizing polymer. Alternatively, where the solvent is alcohol, any suitable alcohol-soluble polymer can be used. In some embodiments for example, the fiberizing polymer can be selected from the group consisting of: polysaccharides (e.g., starch, chitosan, N-[(3'-hydroxy-2',3'-dicarboxy)ethyl]chitosan, dextran, and cellulosic polymers including cellulose ether, isopropyl cellulose, hydroxyethyl, hydroxylpropyl cellulose, cellulose acetate, cellulose nitrate, cellulose ethyl ether, cellulose ethyl hydroxyethyl ether, and cellulose methyl hydroxyethyl ether); gums (e.g., guar gum compounds, konjac glocomannan, pullulan, xanthan gum, i-carrageenan, alginates, alginic ammonium salts); polyacrylates (e.g., polyacrylic acid, poly(methacrylic acid), poly(2-hydroxyethyl acrylate), poly(2-(dimethylamino)ethyl methacrylate-co-acrylamide), poly(1-glycerol methacrylate), poly(2-hydroxyethyl methacrylate/methacrylic acid) 90:10, poly(2-hydroxypropyl methacrylate), poly(2-methacryloxyethyltrimethylammonium bromide), poly(ethyl acrylate/acrylic acid), poly(n-butyl acrylate/2-methacryloxyethyltrimethylammonium bromide), poly(3-chloro-2-hydroxypropyl-2-methacryloxyethyldimethylammonium chloride, dimethyl sulfatequaternary, and poly(ethylene/acrylic acid) 92:8); polyacrylamides and hydrolyzed polyacrylamides (e.g., poly(N-isopropylacrylamide), poly(2-acrylamido-2-methyl-1-propanesulfonic acid),80:20, poly(acrylamide/acrylic acid), poly(acrylamide/2-methacryloxyethyltrimethylammonium bromide), poly(N-iso-propylacrylamide), poly(dimethyldodecyl(2-acrylamidoethyly) ammonium bromide)); polyamines (poly(vinyl amine), poly(4-amino-sulfo-aniline), polyethylene imine, poly(allylamine hydrochloride), polyaniline, poly(g-glutamic acid), poly(2-N-methylpyridiniummethylene iodine), poly(2-ethyl-2-oxazoline), poly[N-(p-sulfophenyl)amino- 3-hydroxymethyl-1,4- phenyleneimino-1,4- phenylene)], poly(benzyltrimethylammonium chloride), and poly(1-lysine hydrobromide)); vinyl and vinylpyridine polymers (e.g., those that can be prepared from vinyl monomers, including, but not limited to, polyvinyl alcohol, poly(vinyl alcohol) 12% acetyl, polyvinylpyrrolidone, poly(vinyl sulfoxide), poly(N-vinyl pyrrolidone-co-vinyl acetate), poly(2-vinylpyridine), poly(4-vinylpyridine N-oxide), poly(4-vinylpyridine), poly(2-vinylpyridine N-oxide), poly(vinyl methyl ether), poly(vinylamine) hydrochloride, poly(vinylphosphonic acid), poly(vinylsulfonic acid) sodium salt, poly(2,4-dimethyl-6-triazinylethylene), poly(3-morpholinylethylene), poly(N-1,2,4-triazolyethylene),poly(methoxyethylene), poly(N-vinylpyrrolidone/2-dimethylaminoethyl methacrylate)); poly(N-propanoyliminoethylene), poly(N-methylpyridinium-2,5-diylethenylene), poly(N-vinylpyrrolidone/vinyl acetate), poly(2-vinyl-1-methylpyridinium bromide), poly(diallyldimethylammonium chloride), poly(oxyethylene) sorbitan monolaurate, poly(4-N-butylpyridiniumethylene iodine), poly(styrenesulfonic acid), N-methyl-4(4'-formylstyryl)pyridinium, methosulfate acetal, poly(allylammonium chloride), poly(allyammonium phosphate), poly(itaconic acid), poly(diallyldimethylammonium chloride), poly(maleic acid), poly(butadiene/maleic acid)); polyethers (e.g., polyethylene oxide, poly(ethylene glycol) bis(2-aminoethyl), poly(ethylene glycol) monomethyl ether, poly(ethylene glycol)-bisphenol A diglycidyl ether adduct, and poly(ethylene oxide-b-propylene oxide)); and copolymers, derivatives, and blends thereof. One particularly preferred (e.g., optional) fiberizing polymer is polyethylene oxide.

The molecular weight of the fiberizing polymer can vary, but is typically greater than about 50,000 amu. Useful polymer molecular weights may vary according to the chemical makeup of the polymer. In embodiments of this disclosure, the fiberizing polymer may be polyethylene oxide with a molecular weight between about 50,000 to 4,000,000 amu (e.g., between about 200,000 amu and 900,000 amu), where the molecular weights are viscosity-average molecular weights. The polyethylene oxide fiberizing polymer may have a number average molecular weight of about 200,000 amu, about 300,000 amu, about 400,000 amu, about 500,000 amu, about 600,000 amu, or about 900,000 amu. The fiberizing polymer preferably (e.g., optionally) has a high solubility in water (or other dispersion solvent), with a solubility of greater than about 0.5 wt % at room temperature being preferred (e.g., optional). It is preferable (e.g., optional) that the fiberizing polymer has an ash content of less than about 5 wt%, when sintered at about 385 °C, with even lower being more preferred.

The amount of fiberizing polymer present in the dispersion can vary. In some embodiments, the dispersion comprises about 1% to about 10% by weight of a fiberizing polymer, based on the total weight of the dispersion. In some embodiments, the weight ratio of fiberizing polymer to PTFE varies. For example, the amount of fiberizing polymer can be about 3.0% to about 5.5% that of the PTFE in the espinning dispersion by weight. The amount of fiberizing polymer required according to the present disclosure may vary depending on the chemical makeup of the polymer.

The espinning dispersion can be formed by any suitable method known in the art. In some embodiments, the PTFE precursor dispersion is mixed with the fiberizing polymer in aqueous solution to form the espinning dispersion or an intermediate dispersion, which is then preferably (e.g., optionally) allowed to homogenize. Although an aqueous mixture is generally used, it is noted that other solvents or dispersion mediums can be used without departing from this disclosure. The dispersion medium or solvent of the fiberizing polymer dispersion may be the same dispersion medium or solvent as that in the PTFE precursor dispersion. The mixing time to form the PTFE espinning dispersion can vary. Generally, the PTFE espinning dispersion is prepared so as to avoid high shear. In one preferred (e.g., optional) method, the polyethylene oxide fiberizing polymer is added to the PTFE precursor dispersion, and then the PTFE precursor dispersion with the polyethylene oxide is allowed to gel slowly, without agitation (for example, over the course of a few days, at which point the formation of a gel layer is apparent), followed by transfer to a jar roller that will turn jars of the PTFE espinning dispersion at a constant rate for several more days. A jar roller is a means to rotate jars to prepare and/or maintain uniformly mixed materials, where the jars are continually rotated at a speed of rotation that can typically be adjusted to ensure that the material is suitably mixed. Exemplary jar rollers are available, for example, from Diemat, Inc., the Mikrons® group, Paul N. Gardner Company, Detroit Process Machinery, and Paul O. Abbe. Any suitable method can be used to produce the PTFE espinning dispersion that results in a material that is generally uniform by visual inspection and that has a suitable viscosity. It is noted that some inhomogeneity in the PTFE espinning dispersion is acceptable; in some embodiments, the PTFE espinning dispersion is filtered prior to spinning. The amount of solvent or other suitable dispersion medium in the PTFE espinning dispersion can be varied to obtain the desired consistency or viscosity.

Additives that are in addition to the fiberizing polymer may be added to the PTFE precursor dispersion, as will be discussed in greater detail below.

In embodiments of this disclosure, the viscosity of the PTFE espinning dispersion is typically within a certain desirable range to allow for the formation of uniform and consistent fibers therefrom. For example, the present disclosure contemplates the use of a PTFE espinning dispersion with viscosities of greater than about 50,000 cP to provide for uniform and consistent fiber formation, as well as faster builds. For example, in some embodiments, the viscosity of the PTFE espinning dispersion is between about 50,000 cP and about 300,000 cP (e.g., about 70,000 cP to about 150,000 cP). Viscosity can be measured, for example, with a Brookfield Viscometer. The desired viscosity of the PTFE espinning dispersion may vary depending on the method of electrospinning to be conducted. For example, an orifice (needle)-based apparatus may require a somewhat higher viscosity than a free surfaced-based apparatus.

It is preferred (e.g., optional) to create a uniform PTFE espinning dispersion that has little to no air trapped in the resulting highly viscous mixture. Accordingly, the PTFE espinning dispersion can be processed to remove entrapped air and to remove clumps or gels by, for example, various mixing and/or filtration protocols.

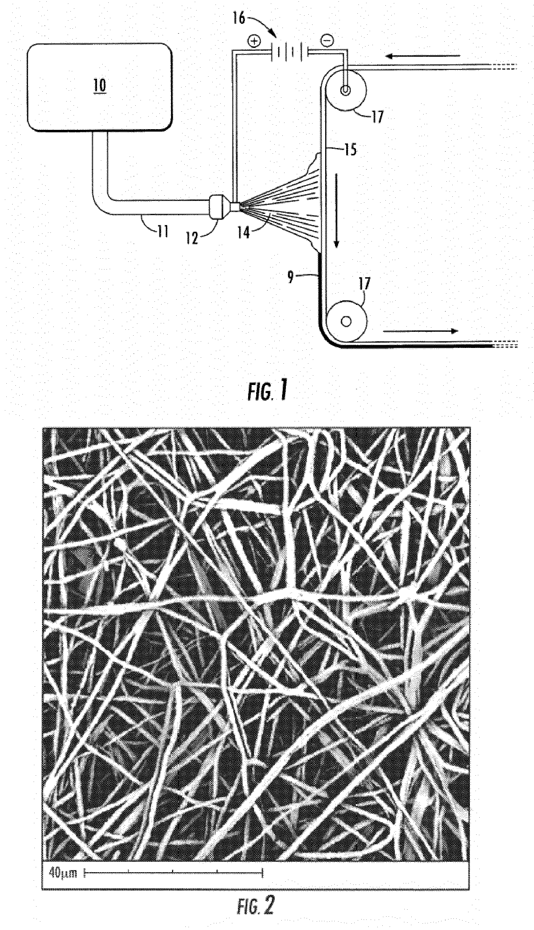

Referring to Fig. 1, a PTFE electrospun material 9 may be formed by electrospinning the PTFE espinning dispersion. In this regard, the PTFE espinning dispersion may be loaded into a controlled pumping device with a fixed conductive element which acts as the charge source 12 for the electrospinning process. The conductive element may have one or several orifices, wherein the PTFE espinning dispersion is discharged through the orifice(s) toward a target, and the orifice(s) and target have opposing electrical charge (or the target may be at ground). Further details regarding apparatus that may be used according to this disclosure are provided, for example, in U.S. Patent Appl. Publ. No. 2010/0193999 to Anneaux et al., which is incorporated herein by reference in its entirety.

Referring more specifically to the electrospinning apparatus schematically illustrated in Fig. 1, a reservoir 10 may be loaded with the PTFE espinning dispersion. A delivery system 11 (e.g., which may include one or more pumping devices) delivers the PTFE espinning dispersion from the reservoir 10 to the charge source 12, which may be, or may include, one or more orifices. The ejection volume from the pumping device is typically set to a predetermined rate that is dependent on the desired configuration of the PTFE electrospun material 9 being made, such as the desired fiber diameters of the PTFE electrospun material. Where an orifice-based system is used, the orifice size is preferably, but not limited to, about 0.01 mm to about 3.0 mm in diameter. A target (or "collection surface") 15 is positioned so that it is spaced apart from the charge source 12. A power source 16, including (but not limited to) a DC power supply, establishes an electrical charge potential between the charge source and the target such that the spun PTFE dispersion 14 is electrically charged opposite the target. The charge source 12 is preferably (e.g., optionally) connected to the positive side of a precision DC power supply 16. The negative side of the power supply 16 is preferably (e.g., optionally) connected to the collection surface or target 15. Although it may not be preferred, the polarity can, in some embodiments, be reversed. It is also possible for the target 15 to be at ground. The spun PTFE dispersion 14 is electrostatically attracted to the target 15 and deposited thereon to form the PTFE electrospun material 9. Although not intended to be limiting, various electrospinning techniques are described, for example, in U.S. Patent Nos. 6,743,273 to Chung et al. and 7,815,427 to Green et al., U.S. Patent Application Publication Nos. 2003/0017208 to Ignatious et al., 2008/0307766 to Petras et al., 2009/0127747 to Green et al., 2009/0199717 to Green et al., 2010/0018641 to Branham et al., and 2011/0111012 to Pepper et al., all of which are incorporated herein by reference in their entirety.

The target/collection surface 15 may be static or in motion (e.g., it may be a continuous or near continuous material that moves through the zone of impact, such as by movement on transport rollers 17, or the like, such that the collection surface may be an endless belt, or the like, of a conveyor). The collection surface 15 can be, for example, a drum (e.g., a cylinder around which an electrospun material can be wrapped) or a sheet. A drum is typically rotated during deposition and the resulting three-dimensional PTFE electrospun material 9 can be used in this "tube"-type form or can be cut to provide the electrospun material in sheet form. A sheet is a flat collection surface with discrete dimensions. In some further embodiments, the collection surface 15 is any material that can be coated, i.e., covered. The shape, size, and geometry of the material that forms the collection surface 15 can vary. For example, the collection surface 15 can be a device, including but not limited to, implantable medical devices (e.g., tissue scaffolding, stents, grafts, and occlusion devices). In such embodiments, the electrospinning is conducted such that the device is covered with the PTFE electrospun material 9. In such embodiments, the device may be coated with another material prior to the electrospinning or the device can be directly covered, such that the PTFE electrospun material 9 forms a covering layer on the device. The collection surface 15 can be any suitable material capable of dissipating electrical charge. In some embodiments, the collection surface 15 can be a metal, ceramic, fiberglass, graphite, or polymeric material (e.g., PTFE or fluorinated polymer). In some embodiments, the material of the collection surface 15 may be selected from stainless steel, copper, cobalt chrome, nickel titanium (e.g., nitinol) and magnesium alloys.

The voltage on the power supply 16 is typically increased to the desired voltage to uniformly draw out the polymer/PTFE espinning dispersion. The applied voltage can vary, but is typically from about 2,000 to about 20,000 volts. The charge induced by the connection of the power supply 16 repels the charged polymer away from the charge source 12 and attracts it to the collection surface 15. The collection surface 15 is preferably (e.g., optionally) placed perpendicular to the orifice system 12 and is moved in at least one direction such that the entire surface is uniformly covered, with the fibers drawn towards the target. The collection surface 15 can, in some embodiments, be rotated to as to ensure coverage on all sides of the collection surface (e.g., where the collection surface comprises a drum or three-dimensional device).

Alternatively, the PTFE espinning dispersion may be electrospun (e.g., into a fabric sheet) using an open bath electrospinning apparatus, as discussed in the following. For example, the open bath electrospinning apparatus can have a wire, cylinder, spike, sharp edge, or similar geometry spinning electrode that creates a perturbation. For the open bath (trough) unit, the ejection volume is dependent upon the viscosity of the dispersion, the conductivity of the dispersion, the surface tension of the dispersion, the distance from bath to target, and the voltage. These factors can also affect the thickness of the fabric and the desired fiber diameter. The charge source is preferably (e.g., optionally) connected to the positive side of a precision DC power supply. The negative side of the power supply is preferably (e.g., optionally) connected to the collection surface. Alternatively, the collection surface can be at ground. The polarity can be reversed but this may not be preferred. The applied voltage can vary, but is typically from about 40,000 volts to about 200,000 volts (e.g., about 80,000 to about 120,000) volts over a typical collection distance of about 100 to about 400 mm.

The charge induced by the connection of the power supply repels the charged polymer away from the charge source and attracts it to the collection surface. In open bath electrospinning, the collection surface is typically a sheet. The sheet surface can be any suitable metal or polymeric material, wherein stainless steel may be a particularly preferred material. The voltage on the power supply is increased to the desired voltage to uniformly draw out the polymer/PTFE espinning dispersion and attract the dispersion to the target. The collection target can be placed above the open bath and is moved in at least one direction such that the entire surface is preferably (e.g., optionally) uniformly covered.

Further details regarding this and other types of apparatus that may be used according to this disclosure and useful parameters for operation of the electrospinning apparatus are provided, for example, in U.S. Patent No. 8,178,030 to Anneaux et al., which is incorporated herein by reference in its entirety. Other types of apparatus and methods of use thereof that can provide electrospun materials for use according to this disclosure are described, for example, in U.S. Patent Nos. 6,743,273 to Chung et al. and 7,815,427 to Green et al., U.S. Patent Application Publication Nos. 2003/0017208 to Ignatious et al., 2008/0307766 to Petras et al., 2009/0199717 to Green et al., 2010/0018641 to Branham et al., and 2011/0111012 to Pepper et al., all of which are incorporated herein by reference in their entirety.

Irrespective of which method is used to form the PTFE electrospun material (e.g., the PTFE electrospun material 9 of Fig. 1), the electrospun material is preferably (e.g., optionally) heated after it adequately covers the collection surface. More specifically and for example, nonwovens prepared by espinning PTFE dispersions typically are sintered to develop the desired properties (e.g., to provide a less tacky, stronger, more durable electrospun material). The electrospun material can be heated in place (i.e., by placing the entire collection surface in an oven) or by removing the electrospun material from the collection surface prior to heating and placing the free electrospun material in an oven. The heating step can serve a number of purposes. It can serve to dry the electrospun material (e.g., by removing the dispersion medium (e.g., a solvent such as water) from the electrospun product). The heating step can also serve to volatilize and remove the fiberizing polymer. The heating step additionally can result in sintering of the PTFE particles. Although not intended to be limiting, it is believed that this sintering step is important to provide the espun PTFE nonwovens with their strength, durability, and other desirable features. The bonding between PTFE particles and/or fibers that is provided by way of sintering may be accompanied by some melting of the PTFE particles and/or fibers. When desired, any such melting may be controlled or otherwise minimized in a manner that seeks to control (e.g., maintain) the porosity of the PTFE nonwoven.

The time and temperature at which the PTFE electrospun material is heated can vary. For example, in typical embodiments, the temperature of the oven is between about 250 °C and about 500 °C (e.g., between about 350 °C and about 485 °C). The time for which the PTFE electrospun material is heated may depend, in part, on the temperature of the oven. The time can also depend on the thickness of the PTFE electrospun material, with thicker electrospun materials typically requiring more time to dry and/or sinter. In some embodiments, the temperature at which the heating/sintering is conducted can depend, in part, upon the characteristics of the optional other components within the gradient structure (which may, for example, not be able to withstand high temperatures that might otherwise be used for PTFE heating/sintering).

In certain embodiments, the samples are generally exposed to heat for a period of time such that at least some (preferably most) of the remaining water is evaporated and at least some (preferably most) of the fiberizing polymer undergoes decomposition and subsequent elimination of the residual material. In some embodiments, the PTFE electrospun material is heated for an hour or less, although longer heating times can be used without departing from this disclosure. In some embodiments, the material is heated for a period of about 30 minutes or less, about 20 minutes or less, about 10 minutes or less, or about 5 minutes or less. For example, in certain embodiments, the heating and/or sintering is conducted for a time of between about 1 and about 30 minutes, preferably between about 5 and about 10 minutes.

The drying, volatilizing, sintering and/or the like can occur simultaneously or in a series of steps. While not intended to be limited by any theory, it is believed that some drying (e.g., removal of the solvent) may occur upon completion of electrospinning. It is further believed that some small degree of fiber rearrangement may occur at this point. Then, when the electrospun material is heated, preferably (e.g., optionally) the majority of the remaining dispersion medium (e.g., solvent) and the fiberizing polymer (e.g., greater than about 80% by weight, preferably (e.g., optionally) greater than about 90% or 95% by weight, and most preferably (e.g., optionally) greater than about 98 or 99% by weight) is removed from the PTFE electrospun material. It is generally understood that espun fabric undergoes shrinkage upon heating. While not limited to any theory, the shrinkage is believed to occur in two steps: the initial drying and fiber rearrangement following the electrospinning process, and the removal of the dispersion medium and fiberizing polymer by heating.

As noted above, the heating can also result in sintering. Sintering refers to the fusion of individual PTFE particles to produce a nonwoven, PTFE-based electrospun material. The sintering of the electrospun material generally results in the formation of a stronger, more durable electrospun material. The level of sintering can vary. During heating, the electrospun material can be monitored to evaluate the sintering level by various methods (e.g., calorimetry). Heating generally causes the electrospun material to undergo physical changes that can be evaluated, e.g., visually. For example, if the electrospun material is visually sticky and still tacky, this generally suggests that the electrospun material should be heated longer. If there is discoloration (e.g., yellowing) of the electrospun material, it commonly indicates the presence (i.e., incomplete decomposition) of the fiberizing polymer and this also suggests that the electrospun material should be heated longer.

The PTFE espun materials are preferably fibrous. Particularly preferred espun fibers have a diameter of at least about 0.01 µm. In a particularly preferred exemplary embodiment, the PTFE espun material, after sintering, has fibers deposited in a density such there is a range of distances of about 0.1 µm to about 50 µm between points of contact between fibers.

### Espun Filtration Media and Preparation Thereof

One aspect of this disclosure is the provision of filtration media comprising, or consisting essentially of, one or more fibrous, nonwoven PTFE espun materials. Such espun PTFE filtration media are beneficial in that, in at least some embodiments, the filtration media may be suitable for use in harsh environments and/or in high temperature applications.

Various testing methods may be used to evaluate the properties of filtration media. For example, filtration efficiency (FE%) and pressure drop (ΔP mm H₂O) for HEPA and ULPA may be determined using a conventional TSI 8130 Auto Filter Tester. In some embodiments, filter media comprising, or consisting essentially of, espun PTFE exhibits high filtration efficiency. For example, in some embodiments of this disclosure, espun PTFE filter media meet the HEPA efficiency standard of at least 99.97% removal (i.e., capture) of 0.3 µm particles, as evaluated using IEST-RP-CC001.3 (1995) (using 0.3 µm particles, 5.3 cm/s velocity, and 100 cm² testing area for a flat sheet and making use of a TSI8130 Auto Filter Tester). The filtration performance characterizes the ability of a filter medium to remove a specified contaminate at a given concentration and a given flow rate, expressed as penetration percentage, and the final pressure drop. The Filtration Efficiency is then calculated by subtracting the Penetration Percentage from 100%.

When a filter is tested on the TSI8130 Auto Filter Tester, two values are taken: the penetration reading and the pressure drop at a specified flow rate. The pressure drop is proportional to the energy required to force air through the filter. Pressure drop is the differential pressure head across the sample at the rated air flow rate, and pressure drop is typically expressed in inches of water. Pressure drop is measured, for example, as part of HEPA or ULPA testing. Pressure drop provides an indication of the energy cost of causing air or water to flow through a filtration media.

In some embodiments, filtrations performed using filter media comprising, or consisting essentially of, espun PTFE exhibit low pressure drop as the material to be filtered passes through the espun PTFE filter media. In some embodiments, espun PTFE mats (e.g., espun PTFE nonwovens) that meet the HEPA efficiency standard exhibit a lower pressure drop when used for filtration than HEPA microglass-based filtration media. For example, in certain specific embodiments, the pressure drop across a PTFE filter medium can be less than about 40 mm H₂O, less than about 35 mm H₂O, less than about 30 mm H₂O, less than about 25 mm H₂O, or less than about 20 mm H₂O.

In some embodiments, espun PTFE mats capable of meeting the HEPA efficiency standard may be thinner than other materials typically used for HEPA filtration (e.g., HEPA microglass-based filtration media). For example, in some embodiments, the espun PTFE mats capable of meeting the HEPA efficiency standard have an average thickness of less than about 300 µm, less than about 200 µm, less than about 150 µm, less than about 100 µm, less than about 80 µm. In certain specific embodiments, the espun PTFE mats capable of meeting the HEPA efficiency standard have an average thickness of less than about 50 µm.

In one aspect of this disclosure, the espun PTFE mats may simultaneously function like surface filters with low flow resistance, and like depth filters having an interconnected porous structure. In this regard and in accordance with at least some embodiments of this disclosure, the espun PTFE filtration media (e.g., espun PTFE nonwoven material) is configured for providing features of both of the two different filtration platforms, surface and depth filtration, and the espun PTFE filtration media synergistically combines the benefits of each. The membrane-like surface filter media feature of the espun PTFE filtration media seeks to provide great filtration efficiency; whereas the three-dimensional interconnected pore structure of the espun PTFE filtration media seeks to provide low flow resistance and, thus, low pressure drops.

The applications of filtration media produced according to the present disclosure can vary. As noted, in some embodiments, the filtration media can be used as HEPA filters. HEPA filters such as those described herein can have applications, for example, to ensure air quality in medical facilities (e.g., in hospitals such as in special procedure rooms (e.g., surgical rooms), delivery rooms, rooms that accommodate patients with airborne communicable diseases, rooms that accommodate patients with immunodeficiency conditions, laboratory facilities, pharmacy facilities, and sterile rooms (e.g., sterile supply rooms)). HEPA filters such as those described herein can also be used to ensure air quality in research facilities (e.g., industrial clean rooms for microelectronic, pharmaceutical, biological, nanomaterial, or chemical research, development, and production), cars, airplanes, and homes; can be used in vacuum cleaners; can be used for analytical gravimetric analysis; and can be used within personal protective equipment (e.g., respirator systems). In some embodiments, HEPA filters may be used for the filtration of any type of particulate matter including non-toxic and/or non-hazardous particulate matter as well as toxic and/or hazardous particulate matter such as, for example, radioactive matter, biohazardous matter, nuclear matter, bacterial, fungal, or viral particulate matter.

The filtration media provided according to the present disclosure can be modified for incorporation within any suitable type of framework or structure to provide a filtration device (i.e., a filter). In some embodiments, a filter has a support structure and an espun mat comprising, or consisting essentially of, espun PTFE supported by (e.g., upon) the support structure, and such a filter may be removably mounted in a forced fluid path (e.g., gas or liquid), as will be discussed in greater detail below. The gas being filtered may be, for example, air.

For example, filtration media (e.g., HEPA filtration media) as described in U.S. Patent Application Publication Nos. 2011/0139008 to Smithies et al., 2010/0218595 to Dikken et al., 2009/0071327 to Latham et al., 2006/0201511 to Freriks, and 2005/0183232 to Wright et al., which are incorporated herein by reference in their entireties, can be replaced with filtration media as described in this disclosure. Depending upon the application, the filtration media may be incorporated into a filtration device in any suitable form (e.g., in flat, single-layered form, folded form, multi-layered form, and/or pleated form).

Advantageously, in some embodiments, the properties of the espun PTFE can be tailored. The ability to tailor the properties of the espun material can be useful in being able to produce a nonwoven PTFE espun material that is suitable for a given type of filtration. Exemplary properties of a PTFE espun nonwoven that can be tailored for such purposes include fiber diameter, density of fiber packing, uniformity of fiber packing, and extent of defects (i.e., non-uniformities) in the nonwoven structure.

There are various ways by which these properties can be controlled and tailored according to the present disclosure. The PTFE espun materials have macroscopic and microscopic properties that can vary depending on the method of preparation thereof (e.g., the parameters of the spinning process). For example, various average fiber diameters of PTFE fibers within the nonwoven espun material can be obtained. There are several ways to control fiber diameter of espun materials that are known to those skilled in the art. Controlling fiber diameter of espun materials is usually related to either the process or material parameters.

One method of controlling fiber diameter is to increase the stretching of the espun material during the spinning process by increasing the espinning dispersion conductivity. For example, increasing the conductivity generally provides a nonwoven having smaller fiber diameters. In turn, decreasing the conductivity of the espinning dispersion generally provides a nonwoven having larger fiber diameters. There are various means by which the conductivity of the espinning dispersion can be modified. For example, in some embodiments, any suitable additive/reagent that dissociates into ions in the dispersion medium (e.g., water) can be added to the dispersion, so long as the reagent that does not detrimentally affect the stability of the dispersion. For example, and in accordance with embodiments of this disclosure, conductive species that can be used for this purpose include, but are not limited to, ammonium or metal salts (chlorides, nitrates, sulfates, carbonates, etc.), weak acids (nitric, formic, acetic, etc.) or weak bases (ammonium hydroxide, pyridine, etc.). One exemplary conductive species that can be used according to this disclosure is ammonium carbonate. In some embodiments, the conductive species is any salt, typically with the exception of oxides and sulfides.

The amount of conductive species to be added can vary. Generally, the amount of conductive species can be that amount necessary to produce the desired change in conductivity of the dispersion. In some embodiments, the increase in conductivity is proportional to the amount of conductive species added to the system. However, the conductivity increase is typically reagent-specific and thus, the amount of conductive species required to produce a dispersion having the desired conductivity will be different for different conductive species. Although not intended to be limiting, in some embodiments of this disclosure, the amount of conductive species added to / included in the dispersion may be between about 0.001% and about 50% by weight, based on the weight of the dispersion. For example, for certain conductive species, the amount of conductive species added to / included in the dispersion is between about 0.01% and about 10% by weight, between about 0.01% and about 5% by weight, between about 0.01% and about 1% by weight, or between about 0.05% and about 1% by weight, based on the weight of the dispersion.

The desired conductivity of the dispersion can vary. In some embodiments, the range of conductivity that provides the proper web uniformity but does not compact the structure is in the range of about 200 to about 1000 µS/cm (e.g., about 300 to about 800 µS/cm). In other embodiments, the conductivity that provides good uniformity without densification of the structure is in the range of from about 50 to about 1000 µS/cm. Conductivity of the dispersions can be measured, for example, using a Mettler Toledo® SevenMulti™ Modular Meter System using an InLab 731® conductivity probe. PTFE dispersions are generally provided with given initial conductivities, which can vary. Further, it is noted that selection of the conductivity of the dispersion may be affected by the PTFE particle size in the dispersion, as will be further described herein. In other words and in accordance with at least some embodiments of this disclosure, conductivity and PTFE particle size typically are be considered in tandem in order to achieve an espun material with a given fiber size.

Modifying the conductivity of the espinning dispersion may affect the resulting nonwoven fabric in other ways as well. In some embodiments, modifying the conductivity may impact the density of packing of the fibers. For example, increasing the conductivity of the espinning dispersion in some embodiments can result in a reduction of average fiber diameter and can also affect the uniformity and/or density of fiber packing. In some embodiments, increasing the conductivity may provide for a somewhat uniform fabric made up of densely packed, compacted fibers. However, while increasing the conductivity normally improves uniformity, it also can compact the web structure, leading to an espun material that does not exhibit desirable filtration capabilities.

The uniformity of fiber packing within the nonwoven and the density of the packing within the nonwoven can be evaluated visually (e.g., with scanning electron microscopy). Another measure of uniformity is pore size uniformity, which can be determined, for example, by comparing the mean pore size and bubble point pore size (largest pore) of an espun material. The density of the fabric can also be determined by comparing the weight in grams per square meter (GSM) with the overall thickness of the espun material (in mils) for a ratio of GSM/mil thickness. In some embodiments, the packing of fibers within a filtration medium comprising, or consisting essentially of, espun PTFE results in a medium having a GSM/mil ratio of about 30 or less, about 25 or less, about 20 or less, about 15 or less, or about 10 or less. In some embodiments, the GSM/mil ratio is between about 8 and about 25, e.g., between about 9 and about 15.

In some embodiments of this disclosure, fiber diameter in a PTFE espun nonwoven can be controlled by selecting a particular average particle size of the PTFE in the precursor PTFE dispersion from which the spinning PTFE dispersion is prepared. When electrospinning PTFE or any other polymeric material from a dispersion, the particle size is considered to be the building block for the fiber structure. Therefore, in some embodiments, by reducing the size of these building blocks (particles), the resulting fibers in the nonwoven will be proportionally reduced in diameter. Correspondingly, in other embodiments, increasing the average particle size may provide for a nonwoven espun material comprising fibers with larger diameters. However, it has been found that generally modifying the PTFE particle size alone is not always a reliable means for producing high quality, defect-free nonwovens, such as are typically required for filtration applications.

In some embodiments of this disclosure, the two parameters of dispersion conductivity and PTFE particle size work together (e.g., hand in hand) in a manner that seeks to provide desired results. In other words, in some embodiments, a predetermined combination of dispersion conductivity and PTFE particle size is provided in order to produce an espun PTFE nonwoven having the desired characteristics. Although either parameter can generally be adjusted independently to provide for a nonwoven with a reduced fiber diameter, these parameters are generally considered in parallel in the present disclosure because, in some embodiments, neither of these parameters considered alone may provide sufficient control over the filtration properties of the resulting PTFE nonwoven fabric. Certain combinations of PTFE particle size and dispersion conductivity may in fact give rise to undesirable macroscale defects and non-uniformities. These defects and non-uniformities are important to consider for applications in filtration. For example, for filtration purposes, it may be desirable to reduce fiber size; however, reducing the fiber size in some embodiments may result in a denser packing of fibers. In such an espun PTFE material, the filter media may have very high flow restriction due to compaction of the fibers during spinning. Such a filter medium would likely exhibit high pressure drops and energy loss during use in filtration.

As noted, reducing PTFE particle size alone may, in some embodiments, reduce the average fiber diameter; and increasing conductivity in the PTFE espinning dispersion alone may, in some embodiments, reduce the average fiber diameter. For example, in one embodiment, reducing the average PTFE particle size in the dispersion from about 230 nm to about 130 nm resulted in a reduction in PTFE fiber diameter in the nonwoven by about 35%. Similarly, increasing the conductivity of a PTFE dispersion with an average particle size of about 230 nm from 250 µS/cm to 600 µS/cm resulted in a reduction in PTFE fiber diameter in the nonwoven by about 15%. Although it is apparent from this data that both particle size and conductivity play a role in impacting fiber diameter of the final nonwoven, it is further apparent that it may be advantageous for these parameters to be considered together.

Various average fiber diameters can be provided by fine tuning both the particle size and conductivity of the PTFE dispersion. In some embodiments, PTFE fiber diameters less than or equal to about 1500 nm, less than or equal to about 1000 nm, less than or equal to about 900 nm, less than or equal to about 800 nm, less than or equal to about 700 nm, less than or equal to about 600 nm, less than or equal to about 500 nm, less than or equal to about 400 nm, less than or equal to about 300 nm, less than or equal to about 200 nm, or less than or equal to about 100 nm can be obtained by providing an appropriate combination of dispersion PTFE particle size and dispersion conductivity. Thus, in some embodiments, the average diameter of the PTFE fibers is within the range of about 250 nm to about 1500 nm (e.g., about 463 nm to about 1500 nm, or between about 1500 nm and three times the average particle size of the PTFE in the espinning dispersion). In some embodiments, the average diameter of the PTFE fibers is between about 400 and about 900 nm.

It is noted that in some embodiments, the electrospinning process described herein may produce a polymeric (e.g., PTFE) mat that is self-supporting and can stand alone (e.g., rather than being used as a coating). However, in some embodiments, the final espun product can comprise other polymers (e.g., as additional polymeric membrane or film layers in the final product or as provided in a dispersion with PTFE, giving a mixed polymeric mat). Such other polymers may or may not comprise electrospun materials. Generally, any polymer that can be placed into solution or dispersion form can be espun. For example, certain suitable materials that can be espun in accordance with the present disclosure include nylons, polyurethanes (PU), polyesters, fluorinated ethylene propylene (FEP), combinations thereof, or the like. Nonwovens prepared by espinning course dispersions (e.g., suspensions) typically are sintered to develop the desired properties; however, nonwovens prepared by espinning from solutions may develop their properties during spinning and drying.

In the following, first through sixth exemplary embodiments are discussed after a discussion of a comparative example of espun PTFE nonwoven fabric. Each of the first through sixth exemplary embodiments may be like the above-discussed embodiments, except for variations noted and variations that will be apparent to one of ordinary skill in the art.

### Comparative Example of Espun PTFE Nonwoven Fabric :

In one aspect of this disclosure, the comparative example described in the following is used for purposes of comparison. In the comparative example, a fiberizing polymer in the form of poly(ethylene oxide) (e.g., polyethylene oxide) (300,000 kDa - 40 grams) was added to 1 L of a precursor dispersion of PTFE particles in water, and then the dispersion was allowed to gel for about 5 days. PTFE particles of the precursor dispersion of the comparative example had an average particle size of about 230 nm. The precursor dispersion of the comparative example was obtained from Daikin Industries, Ltd. as Daikin DX-9025. The gelled dispersion was rolled to combine (about 10 rpm), such as with a jar roller as discussed above, to combine for at least 48 hours to produce a viscous, off-white PTFE espinning dispersion. The conductivity of the PTFE espinning dispersion was approximately 250 µS/cm.

The PTFE espinning dispersion was loaded into a trough where a rotating cylindrical roller was used as the charging electrode and was coated with the PTFE espinning dispersion. A 0.002" thick stainless steel foil sheet (15.5" x 17.5") was mounted on a conductive fabric. The stainless foil was passed into the espinning chamber and used as a collection surface, upon which the PTFE electrospun material was deposited. The collection distance used was approximately 230 cm and the voltage used was 80 kV. Thereafter, the PTFE electrospun material was sintered to form the espun PTFE nonwoven fabric of the comparative example.

As shown in Fig. 2, the resulting espun PTFE nonwoven fabric of the comparative example was fibrous in nature, with the plurality of fibers being configured to define a plurality of pores (e.g., tortuous pathways) that extend through the espun nonwoven fabric. The fibers produced had an average fiber diameter of 1003 ± 105 nm, evaluated by averaging the measurements of at least 50 fibers by using software, namely ImageJ® software. The typical properties of the espun PTFE nonwoven fabrics (e.g., mats) of the comparative example are shown below in Table 1, which gives data for two exemplary PTFE preparations using the same parameters, but targeting different thicknesses for comparison.

**TABLE 1: Comparison of nonwoven PTFE materials of the comparative example**

| Sample | 1 | 2 |
|---|---|---|
| Thickness (mils) | 1.2 | 1.4 |
| Basis Weight (g/m²) | 9.4 | 14.6 |
| Mean Flow Pore (microns) | 4.2 | 3.1 |
| Bubble Point (microns) | 7.2 | 5.1 |
| Gurley number (s/100cc) | 1.2 | 2.4 |
| Viscosity of Spinning Dispersion (cP) Brookfield# | 97,000 | 104,000 |

| | | |
|---|---|---|
| #Measurement taken at an RPM of 2.5 with an S-25 spindle | | |

### First Exemplary Embodiment:

In a first exemplary embodiment of this disclosure, a fiberizing polymer in the form of poly(ethylene oxide) (e.g., polyethylene oxide) (300,000 kDa - 40 grams) was added to 1 L of a precursor dispersion of PTFE particles in water, and then the dispersion was allowed to gel for about 5 days. PTFE particles of the precursor dispersion of the first exemplary embodiment had an average particle size of about 230 nm. The precursor dispersion of the first exemplary embodiment was obtained from Daikin Industries, Ltd. as Daikin DX-9025. A conductive species in the form of ammonium hydroxide (provided as a 28-33% solution by weight in water) was added to the gelled dispersion to produce a final concentration of 5% ammonium hydroxide by volume. The gelled dispersion was rolled to combine (about 10 rpm), such as with a jar roller as discussed above, for at least 48 hours to produce a viscous, off-white PTFE espinning dispersion. The conductivity of the PTFE espinning dispersion was approximately 600 µS/cm.

The PTFE espinning dispersion was loaded into a bath where a rotating cylindrical roller was used as the charging electrode and was coated with the PTFE espinning dispersion. A 0.002" thick stainless steel foil sheet (15.5" x 17.5") was mounted on a conductive fabric. The stainless foil was passed into the espinning chamber and used as a collection surface, upon which the PTFE electrospun material was deposited. The collection distance used was approximately 230 cm and the voltage used was 80 kV. Thereafter, the PTFE electrospun material was sintered to form the espun PTFE nonwoven fabric of the first exemplary embodiment.

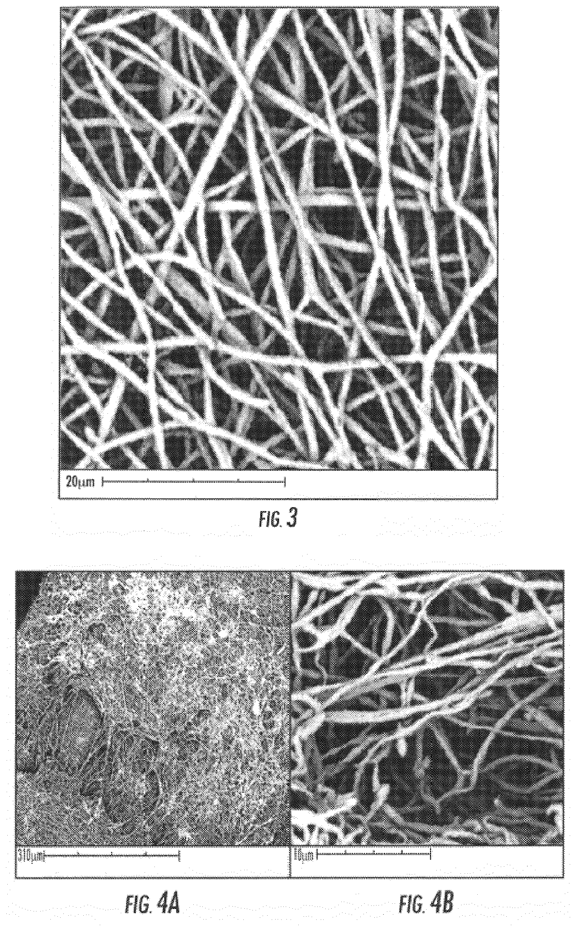

As shown in Fig. 3, the resulting espun PTFE nonwoven fabric of the first exemplary embodiment was fibrous in nature, with the plurality of fibers being configured to define a plurality of pores (e.g., tortuous pathways) that extend through the espun nonwoven fabric. The fibers produced had an average fiber diameter of 852 ± 141 nm (e.g., about 850 nm), evaluated by averaging the measurements of at least 50 fibers by using software, namely ImageJ® software. The typical properties of the espun PTFE nonwoven fabrics (e.g., mats) of the first exemplary embodiment are shown below in Table 2, wherein different thicknesses are provided for comparison.

**TABLE 2: Comparison of nonwoven PTFE materials of the first exemplary embodiment**

| Sample | 1 | 2 | 3 |
|---|---|---|---|
| Thickness (mils) | 0.8 | 1.6 | 2.5 |
| Basis Weight (g/m²) | 8.2 | 16.1 | 28.1 |
| Mean Flow Pore (microns) | 2.9 | 2.3 | 1.8 |
| Bubble Point (microns) | 4.4 | 3.3 | 3.0 |
| Gurley number (s/100cc) | 1.6 | 3.7 | 6.8 |
| Viscosity (cP) Brookfield# | 104,000 | | |

| | | | |
|---|---|---|---|
| #Measurement taken at an RPM of 2.5 with an S-25 spindle | | | |

### Second Exemplary Embodiment:

In a second exemplary embodiment of this disclosure, a fiberizing polymer in the form of Poly(ethylene oxide) (e.g., polyethylene oxide) (300,000 kDa - 40 grams) was added to 1 L of a precursor dispersion of PTFE particles in water, and then the dispersion was allowed to gel for about 5 days. PTFE particles of the precursor dispersion of the second exemplary embodiment had an average particle size of about 160 nm. The precursor dispersion of the second exemplary embodiment was obtained from 3M™ as Dyneon™ TF 5032Z. The gelled dispersion was rolled to combine (about 10 rpm) for at least 48 hours to produce a viscous, off-white PTFE espinning dispersion. The conductivity of the PTFE espinning dispersion was approximately 1200 µS/cm.

The PTFE espinning dispersion was loaded into a bath where a rotating cylindrical roller was used as the charging electrode and was coated with the aforementioned dispersion. A 0.002" thick stainless steel foil sheet (15.5" x 17.5") was mounted on a conductive fabric. The stainless foil was passed into the espinning chamber and used as a collection surface, upon which the PTFE electrospun material was deposited. The collection distance used was approximately 180 cm and the voltage used was 60-70 kV. Thereafter, the PTFE electrospun material was sintered to form the espun PTFE nonwoven fabric of the second exemplary embodiment.

As shown in Figs. 4A and 4B, the resulting espun PTFE nonwoven fabric of the second exemplary embodiment was fibrous in nature, with the plurality of fibers being configured to define a plurality of pores (e.g., tortuous pathways) that extend through the espun nonwoven fabric. The fibers produced had an average fiber diameter of 463 ± 113 nm, evaluated by averaging the measurements of at least 50 fibers by using software, namely ImageJ® software. The typical properties of the espun PTFE nonwoven fabrics (e.g., mats) of the second exemplary embodiment are shown below in Table 3, wherein different thicknesses are provided for comparison.

**TABLE 3: Comparison of nonwoven PTFE materials of the second exemplary embodiment**

| Sample | 1 | 2 |
|---|---|---|
| Thickness (mils) | 1.3 | 0.9 |
| Basis Weight (g/m²) | 41.9 | 36.7 |
| Mean Flow Pore (microns) | 1.6 | 1.6 |
| Bubble Point (microns) | 4.6 | 2.9 |
| Viscosity (cP) Brookfield# | 107,000 | 147,000 |

| | | |
|---|---|---|
| #Measurement taken at an RPM of 2.5 with an S-25 spindle | | |

### Third Exemplary Embodiment:

In a third exemplary embodiment of this disclosure, a fiberizing polymer in the form of Poly(ethylene oxide) (e.g., polyethylene oxide) (300,000 kDa - 40 grams) was added to 1 L of a precursor dispersion of PTFE particles in water, and then the dispersion was allowed to gel for about 5 days. PTFE particles of the precursor dispersion of the third exemplary embodiment had an average particle size of about 130 nm. The precursor dispersion of the third exemplary embodiment was obtained from Daikin Industries, Ltd. as RD-V4. The gelled dispersion was rolled to combine (about 10 rpm), such as with ajar roller as discussed above, for at least 48 hours to produce a viscous, off-white PTFE espinning dispersion. The conductivity of the PTFE espinning dispersion was approximately 300 µS/cm.

The PTFE espinning dispersion was loaded into a reservoir and delivered to a wire-type free surface electrode for spinning. A 0.002" thick stainless steel foil sheet (15.5" x 17.5") was mounted on a conductive fabric. The stainless foil was passed into the espinning chamber where the PTFE electrospun material was deposited. The collection distance used was approximately 260 cm and the voltage used was about 100 kV. Thereafter, the PTFE electrospun material was sintered to form the espun PTFE nonwoven fabric of the third exemplary embodiment.

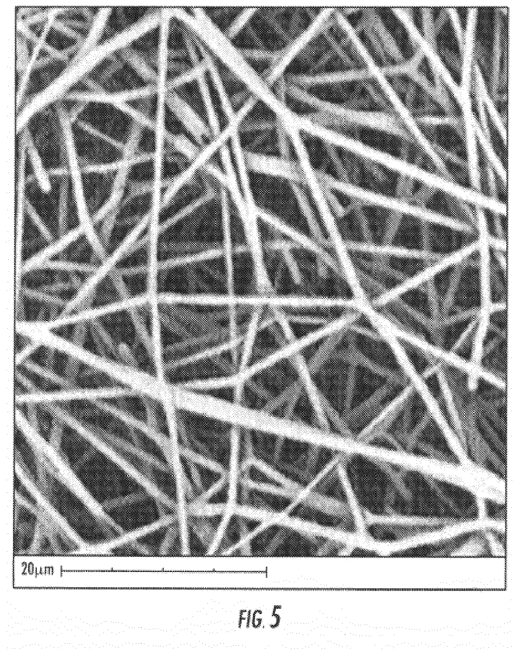

As shown in Fig. 5, the resulting espun PTFE nonwoven fabric of the third exemplary embodiment was fibrous in nature, with the plurality of fibers being configured to define a plurality of pores (e.g., tortuous pathways) that extend through the espun nonwoven fabric. The fibers produced had an average fiber diameter of 651 ± 111 nm (e.g., about 650 nm), evaluated by averaging the measurements of at least 50 fibers by using software, namely ImageJ® software. The typical properties of the espun PTFE nonwoven fabrics (e.g., mats) of the third exemplary embodiment are shown below in Table 4, wherein different thicknesses are provided for comparison.

**TABLE 4: Comparison of nonwoven PTFE materials of the third exemplary embodiment**

| Sample | 1 | 2 | 3 |
|---|---|---|---|
| Thickness (mils) | 0.9 | 2.0 | 2.6 |
| Basis Weight (g/m2) | 13.2 | 23.3 | 30.1 |
| Mean Flow Pore (microns) | 2.0 | 1.9 | 1.7 |
| Bubble Point (microns) | 2.8 | 2.7 | 2.6 |
| Gurley number (s/100cc) | 5.0 | 8.1 | 10.2 |
| Viscosity (cP) Brookfield# | 82,000 | | |

| | | | |
|---|---|---|---|
| #Measurement taken at an RPM of 2.5 with an S-25 spindle | | | |

### Fourth Exemplary Embodiment:

In a fourth exemplary embodiment of this disclosure, a fiberizing polymer in the form of Poly(ethylene oxide) (e.g., polyethylene oxide) (300,000 kDa - 40 grams) was added to 1 L of a precursor dispersion of PTFE particles in water, and then the dispersion was allowed to gel for about 5 days. PTFE particles of the precursor dispersion of the fourth exemplary embodiment had an average particle size of about 80 nm. The precursor dispersion of the fourth exemplary embodiment was obtained from Daikin Industries, Ltd. as RD-V5. The gelled dispersion was rolled to combine (about 10 rpm), such as with ajar roller as discussed above, for at least 48 hours to produce a viscous, off-white PTFE espinning dispersion. The conductivity of the PTFE espinning dispersion was approximately 80 µS/cm.

The PTFE espinning dispersion was loaded into a reservoir and delivered to a wire-type free surface electrode for spinning. A 0.002" thick stainless steel foil sheet (15.5" x 17.5") was mounted on a conductive fabric. The stainless foil was passed into the espinning chamber where the composite PTFE fibers were deposited. The collection distance used was approximately 260 cm and the voltage used was about 100 kV. Thereafter, the PTFE electrospun material was sintered to form the espun PTFE nonwoven fabric of the fourth exemplary embodiment.

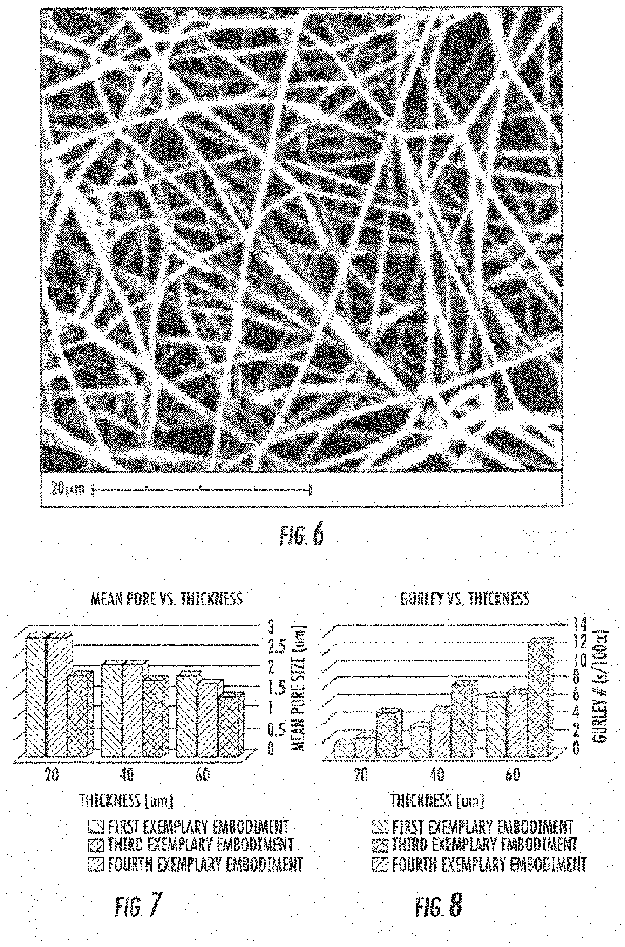

As shown in Fig. 6, resulting espun PTFE nonwoven fabric of the fourth exemplary embodiment was fibrous in nature, with the plurality of fibers being configured to define a plurality of pores (e.g., tortuous pathways) that extend through the espun nonwoven fabric. The fibers produced had an average fiber diameter of 609 ± 86 nm (e.g., about 550 nm), evaluated by averaging the measurements of at least 50 fibers by using software, namely ImageJ® software. The typical properties of the espun PTFE nonwoven fabrics (e.g., mats) of the fourth exemplary embodiment are shown below in Table 5, wherein different thicknesses are provided for comparison.

**TABLE 5: Comparison of nonwoven PTFE materials of the fourth exemplary embodiment**

| Sample | 1 | 2 | 3 |
|---|---|---|---|
| Thickness (mils) | 1.2 | 1.8 | 2.4 |
| Basis Weight (g/m²) | 11.8 | 20.4 | 26.2 |
| Mean Flow Pore (microns) | 2.9 | 2.3 | 1.8 |
| Bubble Point (microns) | 4.4 | 3.3 | 3.0 |
| Gurley number (s/100cc) | 2.9 | 5.5 | 7.4 |
| Viscosity (cP) Brookfield# | 92,700 | | |

| | | | |
|---|---|---|---|
| #Measurement taken at an RPM of 2.5 with an S-25 spindle | | | |

### Fifth Exemplary Embodiment:

In a fifth exemplary embodiment of this disclosure, a fiberizing polymer in the form of poly(ethylene oxide) (e.g., polyethylene oxide) (300,000 kDa - 40 grams) was added to 1L of a precursor dispersion of PTFE particles in water, and then the dispersion was allowed to gel for about 5 days. PTFE particles of the precursor dispersion of the fifth exemplary embodiment had an average particle size of about 160 nm. The precursor dispersion of the fifth exemplary embodiment was obtained from Daikin Industries, Ltd. as RD-V7. The gelled dispersion was rolled to combine (about 10 rpm), such as with ajar roller as discussed above, for at least 48 hours to produce a viscous, off-white PTFE espinning dispersion. A conductive species in the form of ammonium hydroxide was added (provided as a 28-33% solution by weight in water) to produce a final concentration of 5% ammonium hydroxide by volume in the espinning dispersion. The conductivity of the espinning dispersion was approximately 450 µS/cm. The espinning dispersion was loaded into a reservoir and delivered to a wire-type free surface electrode for spinning. A 0.002" thick stainless steel foil sheet (15.5" x 17.5") was mounted on a conductive fabric. The stainless foil was passed into the espinning chamber where the composite PTFE fibers were deposited. The collection distance used was approximately 260 cm and the voltage used was about 100 kV. Thereafter, the PTFE electrospun material was sintered to form the espun PTFE nonwoven fabric of the fifth exemplary embodiment.

The resulting non-woven fabric of the fifth exemplary embodiment was fibrous in nature, with the plurality of fibers being configured to define a plurality of pores (e.g., tortuous pathways) that extend through the espun nonwoven fabric. The fibers produced had an average fiber diameter of 690 ± 117 (e.g., about 650 nm). The typical properties of the espun PTFE nonwoven fabrics (e.g., mats) of the fifth exemplary embodiment are shown below in Table 6, wherein different thicknesses are provided for comparison.

**TABLE 6: Comparison of nonwoven PTFE materials of the fifth exemplary embodiment**

| Sample | 1 | 2 | 3 |
|---|---|---|---|
| Thickness (mils) | 0.9 | 1.4 | 2.4 |
| Basis Weight (g/m²) | 10.6 | 16.4 | 29.2 |
| Mean Flow Pore (microns) | 2.2 | 2.0 | 1.7 |
| Bubble Point (microns) | 2.7 | 2.5 | 2.1 |
| Gurley number (s/100cc) | 4.2 | 6.8 | 13.2 |
| Viscosity (cP) Brookfield# | 92,700 | | |

| | | | |
|---|---|---|---|
| #Measurement taken at an RPM of 2.5 with an S-25 spindle | | | |

### Sixth Exemplary Embodiment:

In a sixth exemplary embodiment of this disclosure, a fiberizing polymer in the form of poly(ethylene oxide) (e.g., polyethylene oxide) (300,000 kDa - 40 grams) was added to 1L of a precursor dispersion of PTFE particles in water, and then the dispersion was allowed to gel for about 5 days. PTFE particles of the precursor dispersion of the sixth exemplary embodiment had an average particle size of about 230 nm. The precursor dispersion of the sixth exemplary embodiment was obtained from Daikin Industries, Ltd. as Daikin DX-9025. The gelled dispersion was rolled to combine (about 10 rpm), such as with a jar roller as discussed above, to combine for at least 48 hours to produce a viscous, off-white PTFE intermediate dispersion. Samples were taken from the intermediate dispersion and various amounts of a salt in the form of ammonium carbonate were added to each sample to create PTFE espinning dispersions, wherein the amounts and respective conductivities of the espinning dispersion are listed in Table 7.

Each of the PTFE espinning dispersions of the sixth exemplary embodiment were separately loaded into 10 mL syringe with a 21 gauge needle attached by a luer-lok fitting. A 0.002" thick stainless steel foil sheet (15.5" x 17.5") was mounted onto a rolling drum. The syringe was set at a flow rate of 0.5 ml/h. The collection distance used was approximately 10 cm and the voltage used was about 15-20 kV. Thereafter, the PTFE electrospun material was sintered to form the espun PTFE nonwoven fabric of the sixth exemplary embodiment.

The resulting non-woven fabrics of the sixth exemplary embodiment were fibrous in nature, with the plurality of fibers being configured to define a plurality of pores (e.g., tortuous pathways) that extend through the espun nonwoven fabric. The resultant average fiber diameter of the fibers produced and other features of the sixth exemplary embodiment are shown in Table 7.

**TABLE 7: Amounts of ammonium carbonate salt included in PTFE espinning dispersions, and other associated properties, in accordance with the sixth exemplary embodiment.**

| Salt in 100 mL dispersion (g) | Viscosity of Dispersion (cPs) | Conductivity of Dispersion (µS/cm) | pH of Dispersion | Resultant Fiber Diameter (nm) |
|---|---|---|---|---|
| 0.05 | 134,000 | 738 | 9.5 | 1080 ± 160 |
| 0.10 | 159,000 | 1283 | 9.5 | 900 ± 140 |
| 0.15 | 161,000 | 1738 | 9.5 | 710 ± 130 |
| 0.20 | 169,000 | 2270 | 9.5 | 620 ± 08 |

The examples above illustrate fiber size control by modulating both the particle size and conductivity. Table 8, below, summarizes findings from the combinations of these parameters, assuming that the process conditions are optimized for each material. Referring to Table 8 and contrasting between the comparative example and the third exemplary embodiment, it can be seen that by generally reducing particle size alone, the average fiber diameter was reduced by about 35% (1003 nm vs. 651 nm). Referring to Table 8 and contrasting the comparative example and the first exemplary embodiment, it can be seen that smaller fibers were obtained by increasing the conductivity while holding the particle size constant. Comparing the second exemplary embodiment to the third and fourth exemplary embodiments also shows that smaller fibers were obtained by increasing the conductivity. This data may indicate, or may suggest, that simply controlling one of the parameters of particle size and conductivity may not be sufficient to provide complete control over fiber size and/or filtration capabilities. At least in theory, it appears reasonable to consider that fiber diameters may be reduced further to below 0.1 µm by having the right combination of particle size and conductivity.

The last column in Table 8 shows a ratio of grams per square meter (GSM) per mil thickness of the fabric and gives an indication of how densely packed the fibers are for each material. Referring to Table 8 and contrasting the comparative example and the first exemplary embodiment, it can be seen that the fibers pack more densely when the conductivity is increased. Furthermore, comparing the second exemplary embodiments to the others in Table 8 demonstrates that the espinning dispersion with the highest conductivity forms a mat in which the fibers are packed considerably more densely than the others in Table 8. In addition to having a GSM/mil ratio of more than 30, the second exemplary embodiment also has the smallest mean pore size as compared to the others in Table 8. For the second exemplary embodiment, the combination of the fiber size and the dense packing of the fibers may result in constricted air flow through the mat, such that the mat may be characterized as being a relatively poor filter material.

**TABLE 8: Characterization of PTFE mats (e.g., espun PTFE nonwovens) formed from various dispersions**

| Example / Embodiment | PTFE Particle Size in Dispersion (nm) | Conductivity of Espinning Dispersion(µS/cm) | Avg. Fiber Diameter in Mat (nm) | Density of Mat (GSM/mil) |
|---|---|---|---|---|
| Comparative Example | 230 | about 250 | 1003 ± 105 | 8.1 |
| First Exemplary Embodiment | 230 | about 600 | 852 ± 141 | 11.4 |
| Second Exemplary Embodiment | 160 | about 1200 | 463 ± 113 | 31.5 |
| Third Exemplary Embodiment | 130 | about 300 | 651 ± 111 | 11.7 |
| Fourth Exemplary Embodiment | 80 | about 80 | 609 ± 86 | 9.6 |
| Fifth Exemplary Embodiment | 160 | about 450 | 690 ± 117 | 12.1 |

Fiber characteristics translate into filtration performance differences. The filtration performance was evaluated using HEPA MIL-STD 282 testing method using 0.3 µm particles, 5.3 cm/s velocity, and a 100 cm² testing area for flat sheets. Table 9 shows a comparison of the thinnest samples of a HEPA Microglass filter (H&V (Hollingsworth and Vose)) and mats of the first, third and fourth exemplary embodiments that meet the HEPA standard efficiency of 99.97%. Mats of the comparative example where unable to meet the HEPA efficiency standard. In contrast, the first exemplary embodiment, with its conductivity modifiers, was able to meet the HEPA efficiency standard. The third and fourth exemplary embodiments, with their relative small particle sizes, also met the HEPA efficiency standard at a reduced pressure drop. However, for exemplary embodiments, a different pressure drop was achieved based on fiber size and packing. Thicker fabrics had to be made for the first and fourth exemplary embodiments than for the third exemplary embodiment to meet the HEPA efficiency standard, producing a higher pressure drop. These findings illustrate that although HEPA efficiency can be met using various techniques there is a strong interplay between particle size and conductivity that dictates the final fabric performance.

**TABLE 9: Filtration characteristics of PTFE mats (e.g., espun PTFE nonwovens) formed from various dispersions**

| Sample | Avg. Thickness (mils) | Frazier Air Perm. (cfm) | FE % | ΔP (mmH₂O) |
|---|---|---|---|---|
| HEPA Microglass | 12.5 | 4.0 | 99.992 | 40.1 |
| First Exemplary Embodiment | 2.4 | 4.7 | 99.996 | 27.5 |
| Fourth Exemplary Embodiment | 1.6 | 6.6 | 99.990 | 22.0 |
| Third Exemplary Embodiment | 1.2 | 8.2 | 99.983 | 15.7 |

The data in Table 9 further shows that exemplary embodiments of espun PTFE mats provided filtration efficiency (FE %) comparable to a standard HEPA Microglass filter (H&V (Hollingsworth and Vose) HEPA Microglass (0.0125" thickness)). However, the espun PTFE mats could be provided in significantly smaller thicknesses than the HEPA microglass, while still providing the desired level of filtration efficiency. In addition, the nonwoven PTFE material of the third exemplary embodiment achieved HEPA filtration efficiency at half (e.g., about half) of the thickness of the nonwoven PTFE material of the first exemplary embodiment.

Additionally, the pressure drop across the 1.2 mil thick piece of espun PTFE of the third exemplary embodiment was significantly less than that produced by the standard HEPA Microglass filter (H&V (Hollingsworth and Vose) HEPA Microglass (0.0125" thickness)). Thus, by modifying the parameters appropriately as provided in the present application, it is possible to obtain espun polymer-based filtration media that are thinner than or comparable in thickness to the HEPA Microglass filter media, and which are capable of functioning as HEPA filters with comparable filtration efficiency with comparable or lower pressure drops as compared with HEPA Microglass filter media.

Regarding data from Table 9, for example and in accordance with an aspect of this disclosure, the 0.0015" thick piece of the espun PTFE of third exemplary embodiment, as compared to the standard HEPA Microglass filter (H&V (Hollingsworth and Vose) HEPA Microglass (0.0125" thickness)):
- is 96% thinner,
- allows greater than twice the air flow therethrough,
- requires 55% less pressure (less ΔP), and
- provides approximately equivalent particle capture efficiency.

In accordance with one aspect of this disclosure, for thickness of the espun PTFE of the third exemplary embodiment ranging from about 0.001" to about 0.0015", each meet the HEPA efficiency standard while requiring less pressure (less ΔP) than the standard HEPA Microglass filter (H&V (Hollingsworth and Vose) HEPA Microglass (0.0125" thickness)), with the reduction in pressure ranging from about 47% to about 61%, and the reduction in pressure being proportionate to the increase in air flow.

Table 10 shows a comparison between the ULPA standard, an ePTFE material, and mats of the third and fifth exemplary embodiments, wherein for the espun PTFE-based materials the comparison is between the thinnest materials that meet the ULPA standard efficiency of 99.999%.

**TABLE 10: Filtration characteristics at ULPA (i.e.. ≥ 99.999% efficiency**

| Sample | Avg. Thickness (mils) | Frazier Air Perm. (cfm) | FE% | ΔP (mmH₂O) |
|---|---|---|---|---|
| ULPA Standard | N/A | N/A | 99.999 | 52.5 |
| ePTFE | 0.3 | 3.5 | 99.999 | 46.9 |
| Third Exemplary Embodiment | 1.9 | 4.7 | 99.999 | 30.7 |
| Fifth Exemplary Embodiment | 1.3 | 5.8 | 100.00 | 22.6 |

The data in Table 10 further shows that espun PTFE mats of exemplary embodiments provided a much lower pressure drop than a standard ULPA material. More specifically, nonwoven PTFE materials of the third and fifth exemplary embodiments having thicknesses of about 50 µm and 35 µm, respectively, met the ULPA standard efficiency of 99.999% at a much lower pressure drop than the ePTFE material.

Regarding data from Table 10, as compared to the 0.0003" thick sample of ePTFE, the 0.0019" thick sample of the espun PTFE of the third exemplary embodiment and the 0.00133" thick sample of the espun PTFE of the fifth exemplary embodiment:
- allow 34% (third exemplary embodiment) and 62% (fifth exemplary embodiment) greater air flow therethrough,
- require 34% (third exemplary embodiment) and 52% (fifth exemplary embodiment) less pressure (less ΔP), and
- provide approximately an equivalent particle capture efficiency.

In accordance with one aspect of this disclosure, for thickness of the espun PTFE of the third exemplary embodiment ranging from about 0.002" (e.g., 0.00019) to about 0.0025", each meet ULPA efficiency requirements while requiring less pressure (less ΔP) than the standard ULPA media, with the reduction in pressure ranging from about 20% to about 48%, and the reduction in pressure being proportionate to the increase in air flow.

Regarding flow characteristics and comparing selected data from Tables 2, 4 and 5 above, Figs. 7 and 8 are graphs (e.g., bar charts) providing comparisons of thickness versus mean pore size and porosity (characterized as the Gurley unit), respectively, for different thicknesses of the nonwoven PTFE materials of the first, third and fourth exemplary embodiments. The Gurley unit is defined as the number of seconds required for 100 cubic centimeters of air to pass through 1.0 square inch of a material at a pressure differential of 4.88 inches of water.

In each of Figs. 7 and 8, from left to right:
- the first, fourth and seventh bars are for the nonwoven PTFE materials of the first exemplary embodiment having thicknesses of 20 µm, 40 µm and 60 µm, respectively;
- the second, fifth and eighth bars are for the nonwoven PTFE materials of the fourth exemplary embodiment having thicknesses of 20 µm, 40 µm and 60 µm, respectively; and
- the third, sixth and ninth bars are for the nonwoven PTFE materials of the third exemplary embodiment having thicknesses of 20 µm, 40 µm and 60 µm, respectively.

As apparent from Figs. 7 and 8, across the range, the espun nonwovens of the third exemplary embodiment espun PTFE, as compared to the espun nonwovens of the first and fourth exemplary embodiments, had relatively smaller pores and higher Gurley numbers due to increased flow resistance. For example, the pore size for the third exemplary embodiment espun PTFE having a thickness of 20 µm is similar to the pore size of the first exemplary embodiment espun PTFE having a thickness of 60 µm. More specifically, the third exemplary embodiment espun PTFE having a thickness of 20 µm has a mean pore size of 1.98 µm, and the first exemplary embodiment espun PTFE having a thickness of 60 has a mean pore size of 1.96 µm. Additionally, the third exemplary embodiment espun PTFE having a thickness of 20 µm has a Gurley number (s/100cc) of 5, whereas the first exemplary embodiment espun PTFE having a thickness of 60 has a Gurley number (s/100cc) of 6.8. Accordingly and regarding the third exemplary embodiment espun PTFE as compared to the first exemplary embodiment espun PTFE, the third exemplary embodiment espun PTFE allows for higher filtration efficiency while allowing for better air flow through the material.

More generally regarding some of the values discussed above and for example, the third exemplary embodiment espun PTFE having a thickness of about 20 µm has a mean pore size of about 1.98 µm and a Gurley number (s/100cc) of about 5, and the first exemplary embodiment espun PTFE having a thickness of about 60 has a mean pore size of about 1.96 µm and a Gurley number (s/100cc) of about 6.8. That is, throughout this Detailed Description section and the figures of this disclosure, measurements or values that are not explicitly specified as being approximate (e.g., "about") may alternatively be approximate (e.g., "about").

As another example regarding the third exemplary embodiment espun PTFE as compared to the first and fourth exemplary embodiments and in accordance with one aspect of this disclosure, the third exemplary embodiment espun PTFE is more (e.g., much more) resistant to cracking during the above-discussed sintering process. This increased resistance to cracking seeks to reduce any problems in the middle of a production run, such as when the electrospinning manufacturing process is incorporated into a continuous production line. As another example regarding the third exemplary embodiment espun PTFE as compared to the first exemplary embodiment espun PTFE and in accordance with an aspect of this disclosure, filtration efficiencies may be achieved with a thinner material; therefore, building up a high thickness of the material may not be required, which may allow for an increase in the speed of the electrospinning manufacturing line. As another example regarding the third exemplary embodiment espun PTFE as compared to the first exemplary embodiment espun PTFE and in accordance with an aspect of this disclosure, a smaller fiber may be produced without densifying the espun fabric, which allows for better filtration properties with a reduced pressure drop. As another example regarding the third exemplary embodiment espun PTFE as compared to the first exemplary embodiment espun PTFE and in accordance with an aspect of this disclosure, the third exemplary embodiment espun PTFE may be more reliable in particle capture testing. As another example regarding the third exemplary embodiment espun PTFE as compared to the fourth exemplary embodiment espun PTFE, the third exemplary embodiment espun PTFE may be less likely to stick to the collection surface 15 (Fig. 1), which seeks to streamline manufacturing.

### Electrospun PTFE Gradient Fabrics

In certain embodiments of this disclosure, two or more layers of espun PTFE fibers may be combined so as to create gradient density fabrics. In certain aspects, the present disclosure provides nonwovens with gradient densities and methods of production and use thereof. Such materials can, in some embodiments, offer a number of advantages over conventional filtration media. For example, it can be possible to tailor certain microscopic properties (e.g., fiber diameter, fiber packing) and macroscopic properties (e.g., pore size, uniformity, density, filtration performance) of the nonwovens described herein.

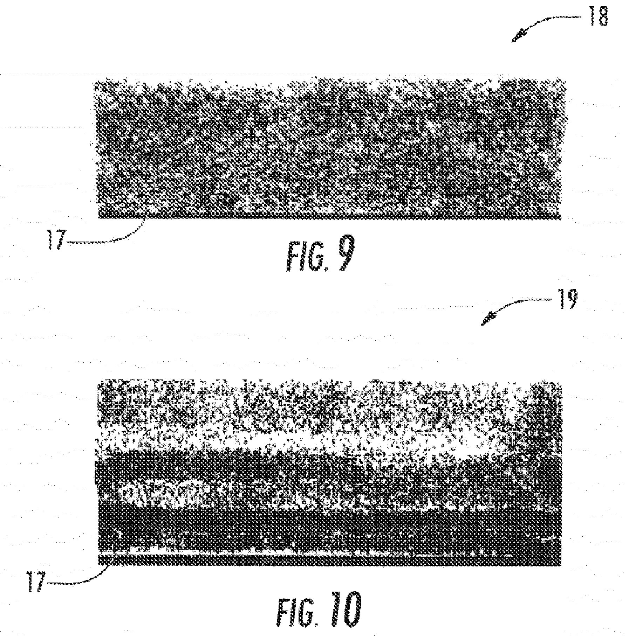

Figs. 9 and 10 are schematic, side cross-sectional views of mats of espun PTFE upon stainless steel foil sheets 17. More specifically, the mat of Fig. 9 (without the foil sheet 17) is in the form of a homogeneous (e.g., substantially homogeneous) espun PTFE filter 18 of constant (e.g., substantially constant) fiber diameter and compacting density (e.g., having substantially the same fiber size throughout the cross section, and having substantially the same density throughout the cross section). In contrast, the mat of Fig. 10 (without the foil sheet 17) is in the form of gradient espun PTFE filter 19 having a variable fiber diameter and a variable compacting density.

For the orientations shown in Figs. 9 and 10, in each of the filters 18, 19, the PTFE fibers were deposited from above, and the direction of air flow through the filter is vertical, from above to below. For the orientation shown in Fig. 10, the gradient filter 19 includes lower and upper sections. As compared to the lower section of the gradient filter 19, the upper section of the gradient filter 19 has larger fibers, lower density and larger pore size. Accordingly, a fluid flowing through the thickness of a gradient filter 19 preferentially passes through the gradient filter's region with larger fiber diameters and less compacting density (larger effective pore size) first, although this disclosure is not intended to be limited to such a direction of flow and may also encompass filters and filtration media wherein the fluid flows in the opposite direction (i.e., first through the region with smaller fiber diameters and greater compacting density (smaller effective pore size first)).

"Gradient density" as used herein is intended to relate to the densities of various layers within a fabric, and refers specifically to a change in the density of the layers within a fabric, when passing from one face of the fabric through the thickness of the fabric to the other face of the fabric (i.e., across the cross-section of the fabric). In certain embodiments, a fabric is thus provided wherein the density of the fabric changes from one face to the other. Whereas this change in density is typically gradual (i.e., with each change in density being of a relatively similar degree through the width of the fabric in preferred embodiments), in some embodiments the change in density from layer to layer is not strictly gradual and one or more large increases and/or decreases in density may exist across the thickness of the fabric. Similarly, in certain embodiments the change in density is regular (i.e., the changes in density occur at regular intervals through the thickness of the fabric), although in some embodiments the changes in density are irregular (i.e., changes in density are not regularly spaced through the thickness of the fabric). It is noted that in certain embodiments, the degree of change across the thickness of the fabric is relatively gradual and/or relatively uniform such that the gradient filter can be thought of as not being strictly "layered" in structure.

In some embodiments, the gradient may be provided by two or more layers with increasing (or decreasing) density, such that each layer across the thickness of the fabric has a higher (or lower) density than the layer before it. In other embodiments, the gradient may provide both increases and decreases in density across the thickness of the fabric. For example, in certain embodiments, the fabric may comprise layers that increase in density across the thickness of a portion of the fabric and one or more layers that decrease in density (e.g., a fabric wherein the layer with the highest density is on the interior of the thickness of the fabric).

The term "layer" as used herein is meant to refer to a unit of a particular average thickness. A layer is not necessarily a discrete and/or uniform thickness. For example, in some embodiments, there may be some degree of intertwining of fibers from various layers. Where the fabric exhibits small areas of intertwining or blending between the layers, these discrete areas may not be considered as gradients. A layer may be continuous or discontinuous along the length and/or width of the fabric. In some embodiment, the gradient fabric of the disclosure can be described as comprising a plurality of layers, e.g., a stack of single layered materials.

The gradient density fabrics of this disclosure can be obtained in various ways. In certain aspects of this disclosure, at least one layer of the gradient density fabric is provided by electrospinning. In certain embodiments, all layers of the gradient density fabric are provided by electrospinning.

In one embodiment, the gradient density is achieved by providing a first mat (which can comprise PTFE or another material) and performing one or more passes of the first mat through an espinning apparatus such that one or more espun layers are deposited on the first mat. The first mat can be provided by electrospinning or by other means. The fiber diameter and density of the deposited fibers and the thickness of the layer can be varied according to the processing parameters used for each pass, as will be described in greater detail herein. Subsequent passes can either provide lesser or greater fiber diameters and/or lesser or greater deposition densities and/or less or greater deposition thicknesses thus achieving the final desired thickness, density, gradient effect, and/or filtration characteristics of the fabric.

In another embodiment, the gradient density is achieved by performing a single pass through multiple espinning zones within an espinning apparatus. The fiber diameters, densities, and thicknesses can be varied according to the processing parameters used for each of the individual zones. Each zone can provide either lesser or greater fiber diameters and/or lesser or greater deposition densities and/or lesser or greater thicknesses thus achieving the final desired thickness, density, gradient effect, and/or filtration characteristics of the fabric. In certain embodiments, a combination of the two approaches described above and/or other approaches for the preparation of gradient fabrics can be used.

In certain embodiments, the gradient fabric consists of a single type of material. By "single type" of material is meant that the chemical makeup of the various layers of the gradient fabric is the same, although the physical properties thereof can vary. For example, a gradient fabric may, in certain embodiments, be made up of two or more electrospun PTFE layers, wherein the layers can have different physical properties, such as different densities, porosities, and/or fiber sizes, etc. It is noted that a given gradient fabric can, in some embodiments, comprise a single type of material, which is exclusive of any substrate, support, etc. on or in which the material is mounted. For example, in certain embodiments, a gradient fabric comprising, or consisting essentially of, a single type of material (e.g., electrospun PTFE) can be laminated to a base layer to provide greater strength. The nature of the substrate, support, etc., can vary. In certain preferred embodiments, the gradient fabric consists essentially of electrospun PTFE and any other component to which it is attached comprises a material that is prepared by means other than electrospinning.

Although some embodiments of this disclosure are directed to espun PTFE, in certain other embodiments, the fabric can include one or more other polymeric layers. In some embodiments, the one or more other layers comprise other electrospun materials. Various polymers that can be placed in a solution have the potential to be espun. Polymer particles that can be made into dispersions (such as PTFE) also can be espun. Espun materials prepared from dispersions are typically sintered to develop the desired properties, but polymers espun from solution generally develop their properties during spinning and drying and thus typically do not require sintering (although the final fabric may be sintered prior to use). Regardless of the composition of the fabric, attachment of the espun layer(s) to each other and/or to other types of layers to produce a fabric for use in filtration generally is accomplished via a sintering process. Therefore, the gradient fabrics are generally in a sintered form for use, e.g., as filtration media.

The gradient fabrics described herein are generally constructed from two or more layers of electrospun PTFE fibers having different properties (e.g., differing fiber sizes and/or differing compacting densities). In some embodiments, a gradient fabric is provided which consists of two layers. However, the number of layers (each comprising a different fiber size and/or a different compacting density than the layers adjacent to it) can vary from two to about fifty (or even greater than fifty in some embodiments). In some exemplary embodiments, the fabric consists of about two to about ten layers.

Each layer of gradient fabrics of this disclosure may be deposited by either a single electrospinning pass or by multiple electrospinning passes. The number of electrospinning passes may, in certain embodiments, be selected based on the desired thickness of the layer. In one embodiment, the electrospun mat may again be passed through the apparatus as discussed previously and a second layer of PTFE fibers (or another type of fiber) of different fiber diameter and/or density and/or thickness deposited upon the initial espun mat. Additional layers of PTFE fibers (or another type of fiber) of different fiber diameters and/or densities and/or thicknesses may be deposited in this fashion until the desired thickness or other fabric characteristics are obtained.

A polymeric PTFE mat directly produced via electrospinning methods is typically fragile and typically must be sintered to provide a sufficiently strong and durable fabric, as discussed above. In some embodiments, the temperature at which the heating/sintering is conducted can depend, in part, upon the characteristics of the optional other components within the gradient structure (which may, for example, not be able to withstand high temperatures that might otherwise be used for PTFE heating/sintering).

In certain embodiments, the polymeric PTFE mats are generally exposed to heat for a period of time such that at least some (preferably most) of the remaining water is evaporated and at least some (preferably most) of the fiberizing polymer undergoes decomposition and subsequent elimination of the residual material.

### Control of Various Properties

In general, filtration media comprising small fibers provide more surface area, and thus can achieve greater filtration efficiency. However, smaller fibers also tend to increase packing density, resulting in higher pressure drops (ΔP), which translates into higher energy consumption, shortened filter life, and high cost in the filtration application. Media comprising large fibers typically provide more physical support, preventing collapse of the filter during use and ensuring good flow characteristics with low ΔP; however, these media may have poor filtration efficiency due to the low surface area. In certain embodiments, the goal of a gradient medium, such as those described herein, is to improve (e.g., significantly improve) the filtration efficiency yet maintain the low ΔP of a larger fiber medium, thus yielding optimal filtration performance.

As indicated, one advantage of the gradient fabrics described herein is that the layers thereof can be tailored so as to provide materials with given physical properties. Tailoring the properties of the individual layers of the fabrics can, in some embodiments, afford gradient fabrics that are useful as filtration media (e.g., which exhibit exceptional pressure drop characteristics, exceptional filtration efficiency, or both).

For example, in certain embodiments, control of the properties of a given layer of the gradient fabric is accomplished by modifying the thickness, fiber diameter, compacting density, and/or web uniformity of the one or more electrospun layers. These parameters and exemplary means for control thereof are discussed in greater detail below. Further, selection of the chemical makeup of the various layers can provide an additional degree of control through the introduction of electrospun fibers of various types of polymers. In some embodiments of this disclosure, it is advantageous to control the fiber diameters of the electrospun PTFE. There are several ways to control fiber diameter, usually related to either the process or material parameters. Reiterating from above, one exemplary means for decreasing fiber diameter is to increase the stretching of the spinning material during the spinning process by increasing the conductivity of the spinning material (i.e., the dispersion or solution). This can be done by adding conductive species that do not detrimentally affect the stability of the dispersion or solution. For example, conductive species that may be useful according to this disclosure include, but are not limited to, ammonium or metal salts (chlorides, nitrates, sulfates, etc.), weak acids (nitric, formic, acetic, etc.), or weak bases (ammonium hydroxide, pyridine, etc.). Reduction of fiber size by increasing conductivity alone can provide very uniform fabric made up of densely packed, fine fibers; however this fabric can have very high flow restriction due to compaction of the fibers during spinning causing high pressure drops and energy loss.

Also reiterating from above, another means for controlling fiber diameter relates to adjusting dispersion particle size. When electrospinning PTFE (or any other polymeric material from a dispersion), the particle size is considered to be the building block for the fiber structure. Therefore, by reducing the size of these building blocks (particles), the fiber diameters of the fibers produced therefrom are proportionally reduced in diameter. However, although fiber size can be reduced by adjusting particle size, an improper level of conductivity will typically not provide high quality filtration media due to macroscale defects and non-uniformities.

Further reiterating from above, either increasing conductivity or reducing particle size can provide a reduction in fiber diameter; however, taken alone, neither of these two methods appears to provide optimal control over the filtration properties of the produced fabric. Therefore, in certain embodiments, these methods for controlling fiber diameter are advantageously used in tandem to provide layers having the desired fiber diameter, with little to no macroscale defects and/or non-uniformities. The relationship between the various parameters of the electrospinning methods and the materials themselves define the ultimate fiber diameters.

Another property of electrospun layers that can advantageously be controlled according to certain embodiments of this disclosure is compaction density. There are several methods to control compaction density, usually related to either the process or material parameters. One method for controlling the compaction density is to adjust the spinning solution conductivity by the addition of conductive species that do not detrimentally affect the stability of the dispersion, as described above. Another method that may be used to control the compaction density is increasing the voltage during espinning, which generally results in greater compaction of the electrospun mat.

Various other methods can be used for achieving greater compaction density. The following are additional embodiments for producing greater or lesser compaction density depositions, which are not intended to be limiting, as any suitable methods known for such purposes can be used. It is to be understood that not all of these techniques are compatible such that some of them may not be performed concurrently to produce higher or lower compaction density fabrics. However, where relevant, one or more of the techniques may be used together, in certain cases, to produce a higher or lower compaction density fabric than might be produced by using a single technique. General guidelines applicable to certain embodiments of this disclosure, and implicated in the electrospinning of the mat include: 1) for a given thickness, a one-pass deposition (i.e., producing a single layer) has higher compacting density than multiple passes (i.e., producing multiple layers) to reach the same thickness; 2) the first electrospun layer deposited upon the target/deposition surface has the highest level of compaction under that set of processing conditions; 3) for multiple passes, the deposition layers have less compacting density as the number of layers increase; 4) as the distance from the target/deposition surface increases, the compacting density decreases; 5) for a given thickness and equivalent number of passes, the longer the time interval between the passes, the lower the compacting density; and 6) the "wetter" the deposition (i.e., the less concentrated the dispersion), the greater the compaction.

In some embodiments, compaction and densification of the fabric can be achieved by processing the fabric after production. For example, the fabric can be calendared using heat and pressure post sintering. As described, electrospun PTFE-based gradient fabrics offer high filtration efficiency with minimal pressure drop in certain embodiments. Even higher efficiencies may be obtained, in some embodiments, by post-sintering compaction of the fabric, such as by calendaring the fabric (although this can, in certain embodiments, result in a corresponding increase in the pressure drop). In another embodiment, layers of different fiber diameters and/or densities may be laminated together under temperature and pressure. This is particularly favorable when using thermoplastic polymer fibers with melting temperatures less than the lamination temperature. In this case the fibers melt and create small pore structures, while adhering the laminates together.

An additional physical parameter that can be adjusted to modify the properties of the material is the uniformity of the electrospun mat. High uniformity on both the macro and micro scales is advantageously. While a random deposition of the fibers during electrospinning is the objective, poor process control can result in the interaction of fibers to form fiber bundles during deposition; it is beneficial to minimize the bundles. Non-random espinning from a cylinder or wire due to insufficient or excess dispersion or congealed dispersion can affect (i.e., decrease or increase) the amount of fiber being spun from a given location on the wire or cylinder and result in an uneven distribution of fibers. In certain embodiments, additives to the dispersion can improve the espinning uniformity by reducing the tendency to create fiber bundles. A reduction in fiber bundles provides a smoother more even surface, and as a result decreases the variation in thickness of the fabric.

Another physical parameter that can be tailored is pore size. Various other parameters (e.g., fiber diameter, density, thickness, and web uniformity of a deposited PTFE espun layer) determine the pore size (and effective pore size) of a layer in the sintered material. Since these attributes of the mat can be controlled as described herein, this provides some degree of control over the pore sizes as well, which further gives some degree of control over the filtration properties of the material. The layered gradient fabrics of this disclosure can, in some embodiments, exhibit the additional desirable characteristics of enhanced filtration efficiency with lower pressure drop than comparable fabrics without a gradient structure.

Other means for modifying certain physical characteristics of a given layer of electrospun material are known and may be employed herein as well. For example, electrospun fibers in direct contact with a substrate (e.g., a metal substrate) can, in some embodiments, flatten, which may reduce the immediate pore size even more. Wetter depositions also can be expected to provide more flattened fibers in certain embodiments. Thus, layers incorporating these attributes often comprise the smallest pore size layers.

As used herein in reference to PTFE and other espun layers, "pore size" is intended to refer to effective pore size (rather than actual pore size) as measured, for example, by air flow and/or water flow (e.g., as defined by ASTM F316 Pore Size Characterization by Bubble Point, which is incorporated herein by this reference). It is noted that, given the nature of the espun fiber mats, it is generally not possible to measure actual pore size of such materials using microscopy methods. The effective pore size of a given espun layer within a gradient fabric of this disclosure can range from about 0.05 µm to about 50 µm. It is noted that, in some embodiments, the effective pore size of a given layer may not be accurately known, such as, for example, where three or more layers are applied and subsequently sintered within the gradient fabric (as in such embodiments, each layer cannot be individually tested). However, the effective pore size of each layer may be approximated by the creation of single layer PTFE electrospun fabric of the same thickness. In some embodiments, the effective pore size of the fabric as a whole may not be reflective of the combination of effective pore sizes of the individual layers.

Due to the layered construction of the gradient fabrics of the present disclosure, one or more of the physical properties of the espun layers as described above can vary from layer to layer within the cross section (thickness) of the fabric. In one exemplary construction, the average pore size changes from large to small based on layer evaluations through the cross section of the gradient fabric.

The ability to control such properties within the individual layers of a gradient fabric of the present disclosure can have implications in designing filtration devices for particular applications. For example, in one embodiment, a high efficiency, low pressure drop, two-layer filter is provided by preparing a filtration system such that the gas or liquid to be filtered first passes through a large pore layer and then through a smaller pore layer. In another embodiment, a filter where pressure equalization is important, yet cross-contamination is not desired is provided, wherein the filtration media comprises three or more layers and may feature, for example, a sandwich construction of outer large pore layers with one or more inner smaller pore layers.

A construction with smaller pore layers as the last layer (i.e., the layer through which the gas or liquid passes last before it leaves the filtration medium) may be most appropriate for a single direction filter. However, this type of filter is not always desired. For example, in some embodiments, other electrospun thermoplastic layers may be incorporated within a filter. Electrospun thermoplastic layers may, in certain embodiments, yield layers with extremely small pore sizes due to the melting of the thermoplastic fibers at PTFE sintering temperatures. Use of such layers in construction of certain filters may be advantageous, such as in embodiments wherein a small pore size is desired in an interior layer of the filter (e.g., where pressure equalization capabilities are desired).

### General Methods and Guidelines

In the following, general methods and guidelines associated with electrospun PTFE gradient constructions are discussed as examples, and not for the purpose of limiting the scope of this disclosure.

For espun PTFE embodiments, the viscosity of the dispersion may be changed by the addition of water or additives containing water, or removal of water from the dispersion without changing the fiberizing polymer to PTFE ratio.

An initial espun polymer layer may be applied at a first desired thickness, typically about 0.5 µm to about 1000 µm, onto a stainless steel surface at a first set of processing and material conditions to give a first espun fiber mat. The deposition can be accomplished using either a single pass or multiple passes. The multiple pass approach may be accomplished by using either a single pass within multiple espinning site apparatus or multiple passes within a single espinning site apparatus.

A second espun polymer layer is then generally applied at a second desired thickness, typically about 0.5 µm to about 1000 µm, onto the first espun fiber mat, but at a second set of processing and material conditions. The deposition can be accomplished using either a single pass or multiple passes as described above. Third, fourth, etc. espun polymer layers, optionally with differing sets of processing and material conditions may be added as desired.

In certain embodiments, the first layer deposited (i.e., the first espun fiber mat) would contain the smallest fiber diameters and/or the highest level of compaction density, although this disclosure is not limited thereto. In some embodiments, the final layer deposited typically would contain the largest fiber diameters and/or the lowest compaction density.

Once the multilayered structure is complete, it is typically placed in an oven at a temperature of between about 35 °C and about 485 °C to allow all layers to bond together. The bonding temperature selection is based on material selection. The fabric thicknesses and the fiber diameters can be determined post sintering.

Various testing methods have been used to evaluate the properties of the fabrics thus produced. For the exemplary embodiments discussed in the following, filtration efficiency (FE%) and pressure drop (ΔP mm H₂O) for HEPA and ULPA testing of the samples is based on a conventional TSI 8130 Auto Filter Tester. The standard method used to determine HEPA capabilities is MIL-STD 282, and the standard method used to determine ULPA capabilities is IES-RP-CC007. Filtration performance at given challenging aerosol and flow rates are based on aerosol generation with NaCl aerosol, having a median diameter of 0.075 µm, geometric standard deviation <1.86, and concentration of 12 - 20 mg/m³. The air velocity for the filtration performance testing is set to 5.3 cm/s and % penetration and ΔP are measured at the end of the test after 1 minute loading. The filtration efficiency is calculated by subtracting the % penetration from 100% efficiency.

Fabric thicknesses are measured using a digital thickness (snap) gauge. Fiber diameters are measured using a Phenom ™ Electron Microscope and by averaging the measurements of at least 50 fibers by using software, namely ImageJ® software.

Basis weights of the fabrics are measured using a balance. The method consists of cutting a fabric sample to a known area and measuring the weight of the fabric sample on a calibrated balance. Although any size standardized die could be used, for espun PTFE basis weight testing purposes, a 3 in² die is used for consistency of method and calculations.

Gurley is measured using a Gurley Densometer test unit. The test method consists of forcing a metered volume of air supplied by a nested cylinder arrangement through a sample held between two plates with known circular orifice areas at low, constant pressure. The fluid reservoir should be partially filled with oil to act as an air seal.

### Seventh Exemplary Embodiment

In a seventh exemplary embodiment, a PTFE espinning dispersion was provided as discussed above with reference to the comparative example, and the PTFE espinning dispersion of the seventh exemplary embodiment had a viscosity of 82,500 cP. The PTFE espinning dispersion of the seventh exemplary embodiment was loaded into a trough where a rotating cylindrical roller was used as the charging electrode and was coated with the PTFE espinning dispersion. A 0.002" thick stainless steel foil sheet (15.5" x 17.5") was mounted on a conductive fabric. The stainless foil was passed into the espinning chamber and used as a collection surface, upon which the PTFE electrospun material was deposited. The collection distance used was approximately 230 cm and the voltage used was 80 kV. A total of 6 passes were made to complete the target (sintered) fabric build of 63 µm.

The PTFE electrospun material was then allowed to dry for two hours followed by sintering at 725 °F for 20 minutes. The resulting homogeneous (e.g., substantially homogenous) non-woven fabric had an average thickness of 65 µm with an average fiber diameter of 883 nanometers, evaluated by averaging the measurements of at least 50 fibers by using software, namely ImageJ® software. The typical properties of the homogeneous espun PTFE nonwoven fabrics (e.g., mats) of the seventh exemplary embodiment are shown below in Table 11.

**TABLE 11: Properties of homogeneous electrospun PTFE nonwoven fabric of the seventh exemplary embodiment**

| | |
|---|---|
| Fiber diameter (nm) | 883 |
| Thickness (µm) | 65 |
| Basis Weight (g/m²) | 21.60 |
| Gurley (s/100 cc) | 2.24 |
| Filtration Efficiency (%) | 96.41 |
| Pressure Drop (mm H₂O) | 10.7 |

### Eighth Exemplary Embodiment

A PTFE espinning dispersion of an eighth exemplary embodiment was prepared like the PTFE espinning dispersion of the seventh exemplary embodiment, except that the PTFE espinning dispersion of the eighth exemplary embodiment further included 25 mL of ammonium hydroxide. The PTFE espinning dispersion of the eighth exemplary embodiment had a conductivity of approximately 480 µS/cm and a viscosity of 107,000 cP. The PTFE espinning dispersion of the eighth exemplary embodiment was loaded into a trough where a rotating cylindrical roller was used as the charging electrode and was coated with the PTFE espinning dispersion. A 0.002" thick stainless steel foil sheet (15.5" x 17.5") was mounted on a conductive fabric. The stainless foil was passed into the espinning chamber and used as a collection surface, upon which the PTFE electrospun material was deposited. The collection distance used was approximately 230 cm and the voltage used was 80 kV. A single pass was made to complete the target (sintered) fabric build of 38 µm.

The PTFE electrospun material was then allowed to dry for two hours followed by sintering at 725 °F for 20 minutes. The resulting homogeneous (e.g., substantially homogenous) non-woven fabric had an average thickness of 56 µm with an average fiber diameter of 774 nanometers, evaluated by averaging the measurements of at least 50 fibers by using software, namely ImageJ® software. The typical properties of the homogeneous espun PTFE nonwoven fabrics (e.g., mats) of the eighth exemplary embodiment are shown below in Table 12.

**TABLE 12: Properties of homogeneous electrospun PTFE nonwoven fabric of the eighth exemplary embodiment**

| | |
|---|---|
| Fiber diameter (nm) | 774 |
| Thickness (µm) | 56 |
| Basis Weight (g/m²) | 25.11 |
| Gurley (s/100 cc) | 3.90 |
| Filtration Efficiency (%) | 99.27 |
| Pressure Drop (mm H₂O) | 15.0 |

### Ninth Exemplary Embodiment:

In a ninth exemplary embodiment, the PTFE espinning dispersion of the eighth exemplary embodiment was loaded into a bath where a rotating cylindrical roller was used as the charging electrode and was rotated and coated with the dispersion. A 0.002" thick stainless steel foil sheet (15.5" x 17.5") was mounted on a conductive fabric. The stainless foil was passed into the espinning chamber where the composite PTFE fibers were deposited. The collection distance used was approximately 230 cm and the voltage used was 80 kV. A single pass was made to complete the target fabric build of 20 µm (sintered).

A bath previously loaded with the PTFE espinning dispersion from Seventh Exemplary Embodiment was then immediately placed within the electrospinning device and the electrospun PTFE fiber coated stainless steel foil passed through the espinning chamber where a second layer of PTFE fibers were deposited. The collection distance used was approximately 230 cm and the voltage used was 80 kV. A single pass was made to complete the target fabric build of 20 µm which combined would yield a total target thickness of approximately 40 µm upon sintering.

The PTFE electrospun material was then allowed to dry for two hours followed by sintering at 725 °F for 20 minutes. The resulting electrospun PTFE gradient non-woven fabric had an average thickness of 48 µm with an average fiber diameter of 774 nm on the bottom side and an average fiber diameter of 883 nm on the top side. The typical properties of the gradient fabric of the ninth exemplary embodiment are shown below in Table 13.

**TABLE 13: Properties of an electrospun PTFE nonwoven gradient fabric of ninth exemplary embodiment**

| | |
|---|---|
| Fiber diameter (nm) | 883 (top)/ 774 (bottom) |
| Thickness (µm) | 48 |
| Basis Weight (g/m²) | 21.44 |
| Gurley (s/100 cc) | 2.62 |
| Filtration Efficiency (%) | 99.89 |
| Pressure Drop (mm H₂O) | 11.17 |

A comparison of the properties of the fabrics of the seventh, eighth and ninth exemplary embodiments is shown in Table 14. The gradient fabric of the ninth exemplary embodiment showed significantly improved filtration efficiency @ 99.89% as compared to 96.41% for the homogeneous large fiber filter medium of seventh exemplary embodiment, and as compared to 99.27% for the homogeneous small fiber filter medium of eighth exemplary embodiment. The gradient filter was constructed and tested such that the air flow was through the large fiber layer first followed by the small fiber layer. This efficiency was achieved even though the gradient fabric had the lowest thickness of the three filtration media tested, i.e., 48 µm as compared to the large and small fiber filters at 65 µm and 56 µm, respectively.

Significantly, the pressure drop for the gradient fabric filter was 11.17 mm as compared to the 10.7 mm and 15.0 mm pressure drops for the large and small fiber filters. Thus, the gradient from large to small fiber provided minimum fiber compaction and more filtration surface area, resulting in high filtration efficiency with a relatively low pressure drop.

**TABLE 14: Comparison of properties of electrospun PTFE nonwoven fabrics of the seventh, eighth and ninth exemplary embodiments**

| Sample | Large Fiber (Seventh Exemplary Embodiment) | Small Fiber (Eighth Exemplary Embodiment) | Gradient Fabric (Ninth Exemplary Embodiment) |
|---|---|---|---|
| Fiber diameter (nm) | 883 | 774 | 883 (top)/ 774 (bottom) |
| Thickness (µm) | 65 | 56 | 48 |
| Basis Weight (g/m²) | 21.60 | 25.11 | 21.44 |
| Gurley (s/100 cc) | 2.24 | 3.90 | 2.62 |
| Filtration Efficiency (%) | 96.41 | 99.27 | 99.89 |
| Pressure Drop (mm H₂O) | 10.7 | 15.0 | 11.17 |

As shown in Table 14, fabrics with smaller fibers exhibit less particle penetration (better efficiency) than fabrics with larger fibers. The fabrics with smaller fibers exhibit a higher pressure drop than observed with fabrics with larger fibers. Thus, to obtain a meaningful quantification of the true performance/efficiency in filtration performance as both the filtration efficiency and pressure drop vary, one cannot rely on either the filtration efficiency or the pressure drop alone. One needs to account for both the filtration efficiency and the pressure drop. One means of doing so is to determine and compare the product of the filtration penetration and the pressure drop. The lower the value for the product of the two the better the performance of the filtration media. This term is referred to as "Filtration Effectiveness" and the comparison for the three filtration media are shown in Table 15. Thus, simply multiplying the penetration by the pressure drop for each fiber diameter provides directly comparable data.

Normally, high values for filtration efficiency correlate with high values for pressure drop and vice versa. It is important to examine the combined effects of the two to evaluate the filtration characteristics of a given material. The best filtration medium would have characteristics of low penetration and low pressure drop. We can model this effect by multiplying the pressure drop by the penetration to obtain the combined effects.

As shown in Table 15, the large fiber medium showed the highest penetration and the lowest pressure drop. While the small fiber medium showed a much reduced penetration (i.e., better filtration efficiency), it also had the highest pressure drop. By providing a gradient medium according to the methods and materials outlined herein, the advantages of both approaches are combined into a single medium. A gradient medium such as described herein can, in some embodiments, be capable of achieving a high level of filtration efficiency, while also maintaining a low pressure drop. One exemplary gradient medium, data for which is shown in Table 15 (Ninth Exemplary Embodiment) clearly exhibits the lowest penetration and a pressure drop that is only slightly higher than that exhibited by the media comprising large fibers (Seventh Exemplary Embodiment). The calculated filtration effectiveness of the gradient fabric is significantly better than that of either the large fiber medium or the small fiber medium. Thus, the gradient medium provided the best filtration effectiveness as compared to the other media.

**TABLE 15: Comparison of Filtration Effectiveness of Electrospun PTFE Nonwoven Gradient Fabrics**

| Sample | Large Fiber (Seventh Exemplary Embodiment) | Small Fiber (Eighth Exemplary Embodiment) | Gradient Fabric (Ninth Exemplary Embodiment) |
|---|---|---|---|
| Fiber diameter (nm) | 883 | 774 | 883 (top)/ 774 (bottom) |
| *Penetration (P) (%) | 3.59 | 0.73 | 0.11 |
| Filtration Efficiency (%) | 96.41 | 99.27 | 99.89 |
| Pressure Drop (mm H₂O) | 10.7 | 15.0 | 11.17 |
| Filtration Effectiveness (P%*ΔP) | 38.41 | 10.95 | 1.23 |

| | | | |
|---|---|---|---|
| * Percent of particles passing through medium. Zero percent Penetration would be 100% Filtration Efficiency. | | | |

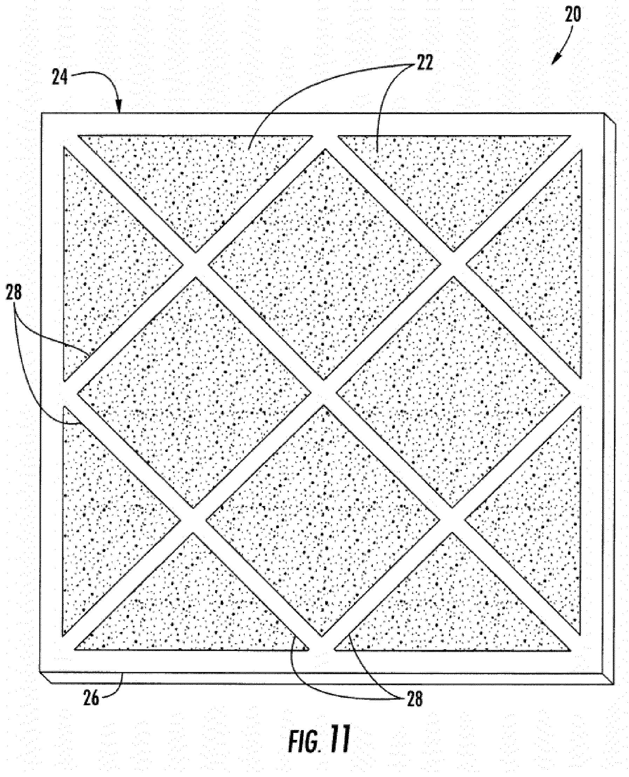

In the foregoing, numerous espun mats, which may more specifically be in the form of espun nonwoven fabrics, are disclosed, and the following reiterates other examples of how espun mats of this disclosure may be used. As mentioned previously, an espun mat (e.g., any one or more of the above-described espun mats, including, but not limited to, the above-discussed espun gradient fabrics) may be a filtration medium of a filter assembly. As a more specific example, Fig. 11 is a schematic pictorial view of a fluid (e.g., gas, or more specifically air) filter 20, in accordance with an embodiment of this disclosure. The filter 20 includes a filtration medium 22 that is at least partially contained by a support structure 24. The filtration medium 22 is schematically illustrated by stippling in Fig. 11. The filtration medium 22 may comprise any one or more of the above-described espun mats (e.g., see Figs. 2-6, 9 and 10), wherein when multiple layers are present they may be superposed with one another such as, but not limited to, as a result of being a gradient fabric. When the filtration medium 22 includes multiple layers, the layers may include one or more of the above-described espun mats in combination with one or more layers of conventional filtration media. The filtration medium 22 may be in any suitable form, such as in flat, single-layered form; folded form; multi-layered form; and/or pleated form.

In the embodiment shown in Fig. 11, the support structure 24 may be a conventional structure that includes a peripheral frame 26 and cross-members 28, and the support structure may be constructed of any suitable material, such as polymeric material, cardboard and/or paperboard. The frame 26 extends at least partially around, or more specifically extends all the way around and encloses, the peripheral edges of the filtration medium 22. Each side of the frame 26 may be in the form of a generally C or U-shaped channel member, wherein the grooves of the channel members are respectively in receipt of marginal portions of the filtration medium 22. The cross-members 28 are connected to the frame 26 and extend across the opposite faces of the filtration medium 22, so that the filtration medium is positioned between opposite groups of the cross-members. In use, a pressure gradient is defined across the filter 20, so that the gas being filtered flows through the openings defined between the cross-members 28 of the upstream group of the cross-members, then through the filtration medium 22, and then through the openings defined between the cross-members 28 of the downstream group of the cross-members. In alternative embodiments, the cross-members may be configured differently or omitted, and similarly the frame 26 may be omitted, configured differently, or replaced with another suitable support structure. For example, it is within the scope of this disclosure for the filtration medium to be configured in any suitable conventional manner for filtration, such as in the form of a cartridge that is removably received in a housing. More generally, the filtration medium of this disclosure may be used in the place of conventional filtration mediums, such as in conventional filter assemblies.

As a specific example of how espun mats of this disclosure may be used in filtration, air filters may be based solely on (e.g., may consist of, or consist essentially of) espun PTFE, or air filters may comprise espun PTFE in combination with other filtration media. In one aspect of this disclosure, advantages of filters comprising, or consisting essentially of, espun PTFE may be based on the combined benefits of being able to adjust the pore structure between the espun PTFE layers and the three-dimensional structure of the espun PTFE. With controlled pore size by adjustments of the fiber size and density, and the tortuous pore path due to the three-dimensional structure, filters comprising, or consisting essentially of, espun PTFE may provide a greater effective surface area for higher filtration efficiency, as compared to at least some conventional filtration media. In addition, the multiple pore pathways through the three-dimensional structure of the espun PTFE filtration media may allow for less pressure drop, as compared to at least some conventional filtration media. These combined factors of the espun PTFE filtration media may lower energy consumption and provide a longer filter life with less frequent filter replacement resulting in less downtime (e.g., in total a significant cost savings for the user), as compared to at least some conventional filtration media. In one aspect of this disclosure, additional benefits of the espun PTFE filtration media may include superior chemical resistance, a wide thermal operating range, exceptionally high flow rates, an inherently hydrophobic material, U.S.P Class VI rating, non shedding characteristics, minimal extractable content from the espun PTFE filtration media, and the ability of the espun PTFE filtration media to capture and retain bacteria.

In at least one embodiment, a heating, ventilation, and air conditioning (HVAC) filter comprising, or consisting essentially of, espun PTFE filtration media of this disclosure (e.g., see Figs. 2-6, 9 and 10, and the filter of Fig. 11) has a Minimum Efficiency Reporting Value (MERV) of thirteen or greater, or more specifically of seventeen or greater, and meets or exceeds HEPA and ULPA ("ultra-low penetration air") filtration efficiency with significantly reduced pressure drop; the pressure drop can be at least 30% lower as compared to at least some conventional filtration media, such as microglass media. In one aspect of this disclosure, an ULPA filter can remove (i.e., capture) from the air at least 99.999% of airborne particles (e.g., dust, pollen, mold and bacteria) with a size of less than 0.3 µm. This efficiency requirement/term characterizes the ability of a filter medium to remove a specified contaminate at a given concentration, expressed as a percent, under specified test conditions.

As another specific example according to at least one embodiment of this disclosure, an industrial dust bag filter constructed of espun PTFE filtration media of this disclosure (e.g., see Figs. 2-6, 9 and 10) compares very favorably with standard media according to ASTM D-6830-02. The espun PTFE filtration media has a filtration efficiency of at least 99.9999% with a minimum 30% reduction in pressure drop. This comparison is with respect to an industrial dust bag filter constructed of expanded poly(tetrafluoroethylene) (ePTFE) material with a filtration efficiency of at least 99.999% .

As another specific example of how espun mats of this disclosure (e.g., see Figs. 2-6, 9 and 10) may be used in filtration, vent filters may be based solely on (e.g., may consist of, or consist essentially of) espun PTFE, or vent filters may comprise espun PTFE in combination with other filtration media. Vent filters may be like (e.g., very similar to) the above-described air filters, with a distinction being that, in use, the passage of the air or gas can go either way through a vent filter. Thus, the goal in using a vent filter is typically to keep two environments separate from one another by way of the vent filter; allowing nothing but the air or gas to enter by way of the vent filter and nothing but the air or gas to escape by way of the vent filter while maintaining constant pressure equilibrium in both environments. In such an application, the vent filter restricts the transport of dust and dirt therethrough, while allowing gas to pass therethrough in response to changing environmental conditions. This passage of gas may be for preventing pressure from building up in a manner that may damage enclosure seals. For example, it is typically desired not to damage enclosure seals, because damaged enclosure seals may expose sensitive components to water and/or debris. As examples, vent filters of this disclosure may be used to restrict the passage of airborne radiological contaminants (e.g., resulting from nuclear fission or fusion), such as in some of the air vented from nuclear power plants. Similarly, vent filters of this disclosure may be used to restrict the passage of airborne diseases. Vent filters of this disclosure may also be used to promote the cleanliness of clean rooms (e.g., rooms in which there is a controlled level of contamination). Clean rooms may be utilized, for example, in the manufacture of electronics, semiconductors, consumer products, and medical devices.

As another specific example of how espun mats of this disclosure (e.g., see Figs. 2-6, 9 and 10) may be used in filtration, liquid filters may be based solely on (e.g., may consist of, or consist essentially of) espun PTFE, or liquid filters may comprise espun PTFE in combination with other filtration media. Similarly to the characteristics for air filters of this disclosure, the ability to control the pore size by fiber diameter and density may provide high efficiency and lower pressure drop for liquid filters of this disclosure as well. In one aspect of this disclosure, the espun PTFE in liquid filters is hydrophobic and inert to a wide variety of solvents, so that liquid filters of this disclosure can be adaptable to a variety of filtration environments. In one aspect, the chemical resistance and high temperature capabilities of espun PTFE allows for the removal of solids from very aggressive solvents and processes. As examples, liquid filters comprising, or consisting essentially of, espun PTFE may be used in chemical processing, beverage processing, and in pharmaceutical, semiconductor and chemical applications.

As another specific example of how espun mats of this disclosure (e.g., see Figs. 2-6, 9 and 10) may be used, separation membranes may be based solely on (e.g., may consist of, or consist essentially of) espun PTFE, or separation membranes may comprise espun PTFE in combination with other filtration media. Separation membranes of this disclosure may be used, for example, in place of evaporation and distillation equipment in industrial applications, such as for improving economics. In accordance with one example of using a separation membrane of this disclosure, each of the separation membrane's / espun PTFE's hydrophobic nature, chemical-resistance characteristics and ability to withstand high temperature are utilized. For example, the separation membrane may restrict the passage of water and polar species therethrough, while allowing for the passage of organic materials therethrough. As discussed above and in accordance with one aspect of this disclosure, the properties of the separation membranes may be controlled by controlling the size of the pores of the espun PTFE that forms or is part of the separation membranes.

In at least one embodiment, a separation membrane comprising, or consisting essentially of, espun PTFE filtration media of this disclosure (e.g., see Figs. 2-6, 9 and 10) may be used as a battery separator. Such a battery separator typically is constructed for being having both uniform and consistent along both its length and width, to have good physical strength with a good puncture strength, and high permeability. More specifically, the puncture strength of the battery separator may be greater than 12 gm/micron, and, regarding permeability, the battery separator may have a MacMullin number in the range of 10 to 12. Similarly, separation membranes constructed of espun PTFE filtration media of this disclosure (e.g., see Figs. 2-6, 9 and 10) may be used in other types of energy storage devices, such as fuel cells.

As another specific example of how espun mats of this disclosure (e.g., see Figs. 2-6, 9 and 10) may be used, engineered fabrics may be based solely on (e.g., may consist of, or consist essentially of) espun PTFE, or engineered fabrics may comprise espun PTFE in combination with other media. The engineered fabrics may be made for a variety of end-use applications. For example, engineered fabrics of this disclosure may be characterized as being performance fabrics and/or protective fabrics. Performance fabrics may provide functional qualities, such as moisture management, repellence of fluids such as water, water resistance, wind resistance, UV protection, anti-microbial qualities and/or thermo-regulation. More specifically and at least in theory, anti-microbial additives may be included in the espun PTFE mats (e.g., espun PTFE nonwovens) of this disclosure, such as for inhibiting degradation of the fabric and controlling odors caused by bacteria.

As another example, espun PTFE mats of this disclosure (e.g., see Figs. 2-6, 9 and 10) may be configured to be, or may be part of, a waterproof breathable material that both prevents (e.g., substantially prevents) penetration of water or other liquids, while allowing the release of moisture vapor and air to provide thermal comfort. In accordance with one aspect of this disclosure, electrospun PTFE has the advantage of providing hydrophobicity and pore size control while allowing for better air permeability, as compared to standard expanded poly(tetrafluoroethylene) (ePTFE) material. The engineered performance fabrics of this disclosure may be used, for example, in the aerospace industry, as wire insulation, for packaging, and/or for temporary enclosures or environments. As another example and as indicated above, engineered fabrics may comprise espun PTFE in combination with other media, and, as a more specific example, in clothing or other fabrics, the espun PTFE may be an outer layer of the clothing or other fabric, and the clothing or other fabric may further include an inner layer (e.g., an innermost layer) of material that is mounted to (e.g., laminated to or otherwise directly or indirectly connected to) the espun PTFE, wherein the inner layer may be operative for drawing perspiration away from the skin of a user, and allowing the perspiration to travel to the one or more outer layers. The inner layer may transfer the moisture by way of capillary action (e.g., wicking). Any suitable, conventional inner layer may be used, such as, but not limited to, synthetic materials such as polyester and microfiber-based fabrics.

The engineered protective fabrics (e.g., espun PTFE mats) of this disclosure (e.g., see Figs. 2-6, 9 and 10) may be used to make clothing for protecting the wearer against hazards caused, for example, by extreme changes in physical environments, dangerous working conditions, and/or human actions. Regarding the espun PTFE mat(s) in such engineered protective fabrics, their three-dimensional structure, controlled pore size and breathability allows for comfort to the wearer while providing protection from environmental hazards. The engineered protective fabrics of this disclosure may be used, for example, in performance apparel, personal protective garments, dust masks, medical textiles and/or medical uniforms. Regarding conventional items comprising conventional fabrics, in those conventional items, one or more of the conventional fabrics may be replaced with the electrospun nonwoven fabrics of this disclosure in situations where such a substitution would be suitable.

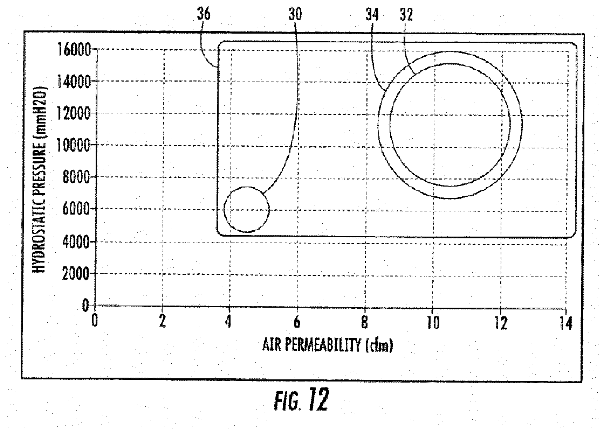

Further regarding the engineered fabrics of this disclosure (e.g., performance fabrics), the espun PTFE may be configured to simultaneously exhibit substantial water resistance and substantial breathability. In this regard, Fig. 12 is a chart illustrating an area 30 identifying a set of data for an espun PTFE nonwoven fabric of the third exemplary embodiment of this disclosure having a thickness of about 3 mils. The 3 mils espun PTFE nonwoven fabric of the third exemplary embodiment was tested, as discussed in greater detail below, and the resulting data from the tests is a statistical sample of the set of data points of area 30. Fig. 12 also includes areas 32, 34, 36 identifying sets of data for embodiments of this disclosure that may be at least partially prophetic.

Regarding the data points of the data sets respectively identified by areas 30, 32, 34, 36, Fig. 12 includes a Cartesian coordinate system in which values of increasing water resistance extend upwardly along the vertical axis, and values of increasing breathability / air permeability extend to the right along the horizontal axis. The water resistance for the 3 mils espun PTFE nonwoven fabric of the third exemplary embodiment was determined using a hydrostatic pressure test to measure the resistance of the fabric to the penetration of water therethrough, wherein hydrostatic pressure was exerted by a column of water that was is in opposing face-to-face contact with, and extended upwardly from, an upper face of the fabric, and the opposite lower face of the fabric was exposed to the ambient atmosphere. The degree of the water resistance was quantified by the height of the column of water (i.e., the hydrostatic pressure in millimeters of water) at which the water penetrated through the fabric. The breathability / air permeability in cubic feet per minute for the espun PTFE nonwoven fabric of the third exemplary embodiment was determined using a differential pressure air permeability test as specified in ASTM D737 (Frazier air permeability test). Frazier air permeability is the inverse of air flow resistance, and it is usually denoted in cfm/ft² at 0.5 inches of water differential pressure.

For ease of understanding and very generally described, it is noted that the chart of Fig. 12 may be characterized as comprising a Venn diagram, or the like, although and for example, the closed peripheral lines respectively defining the areas 30, 32, 34, 36 of Fig. 12 are not limited to simple closed curves (e.g., whereas the closed peripheral lines respectively defining the areas 30, 32, 34 are generally circular, or more specifically they are each in the form of a circle, in contrast the closed peripheral line defining the area 36 is generally rectangular, or more specifically it is a rectangle with rounded corners). For the Cartesian coordinate system shown in Fig. 12, each of the areas 30, 32, 34, 36 defines a set of data that includes all of the data points that are in that area. In one aspect of this disclosure, for each area 30, 32, 34, 36, the area includes both the set of data points that are coincident with the endless peripheral line that defines the area and the data points that are circumscribed by the endless peripheral line that defines the area (i.e., the area does not include the data points outside of the endless peripheral line that defines the area). In another aspect of this disclosure, for each area 30, 32, 34, 36, the area only includes the set of data points that are circumscribed by the endless peripheral line that defines the area (i.e., the area includes neither the data points coincident with the endless peripheral line that defines the area nor the data points outside of the endless peripheral line that defines the area).

With regard to Fig. 12, one aspect of this disclosure is the provision of espun PTFE nonwoven fabric having a sufficiently high air permeability that seeks to allow the espun PTFE nonwoven fabric to be tailored toward having increased water resistance while maintaining breathability. At least partially prophetically, it is within the scope of this disclosure for espun PTFE nonwoven fabric to have ranges of water resistance and/or air permeability that fall within any one or more of areas 30, 32, 34, 36 in Fig. 12. For example and at least partially prophetically, espun PTFE nonwoven fabric(s) of embodiment(s) of this disclosure may have a water resistance ranging from the lowest water resistance value included in any one of the areas 30, 32, 34, 36 to the highest water resistance value included in any one of the areas 30, 32, 34, 36 and/or an air permeability ranging from the lowest air permeability value included in any one of the areas 30, 32, 34, 36 to the highest air permeability value included in any one of the areas 30, 32, 34, 36, and any other suitable ranges (e.g., within the areas 30, 32, 34, 36) are within the scope of this disclosure. Notwithstanding the foregoing, the areas 30, 32, 34, 36 of Fig. 12 are presented as examples, and other areas, data sets and/or ranges are within the scope of this disclosure.

At least partially reiterating from above, the engineered protective fabrics (e.g., espun PTFE mats) of this disclosure (e.g., see Figs. 2-6, 9 and 10) may be configured to simultaneously exhibit substantial liquid barrier properties and substantial breathability. Accordingly, these fabrics may be used, for example, in medical surgical drapes and drape accessories, and protective apparel such as surgical gowns, isolation gowns, and the like. In accordance with one aspect of this disclosure, unlike other nano fabrics made from other polymers such Nylon and Polypropylene, whether from melt blowing or espinning, at least some of the espun PTFE nonwoven fabrics (e.g., mats) of this disclosure have demonstrated much greater hydro head (e.g., better barrier properties). At the same time, at least some of the espun PTFE nonwoven fabrics of this disclosure have maintained higher air permeability as compared to ePTFE membranes, for providing better comfort and breathability.

Table 16 illustrates comparisons between blood penetration data for comparative examples and exemplary embodiments of this disclosure. Generally regarding the data shown in Table 16, liquid barrier testing is classified in four levels by ANSI/AAMI PB70:2012, "Liquid barrier performance and classification of protective apparel and drapes intended for use in health care and facilities". Two of the levels specified by ANSI/AAMI PB70:2012 require different levels of water impact penetrations and hydrostatic pressures according to AATCC Test Method 127. For Table 16, the data for the hydrostatic pressures (i.e., hydro head) was acquired in accordance with AATCC Test Method 127. For surgical drapes and drape accessories, one of the levels specified by ANSI/AAMI PB70:2012 requires passage of the test identified by ASTM F1670, "Resistance to penetration by synthetic blood". For Table 16, the "blood penetration" data was acquired in accordance with ASTM F1670, "Resistance to penetration by synthetic blood".

**TABLE 16: Comparisons Between Comparative Examples and Exemplary Embodiments for Blood Penetration Testing**

| Comparative Example / Embodiment | Thickness (µ) | Air Perm (cfm) | Hydro Head (cm) | Blood Penetration (pass/fail) |
|---|---|---|---|---|
| Comparative ePTFE membrane | unknown | 3.5 | > 1,000 | pass |
| Comparative spunbond/3 layers meltblown/spunbond | unknown | 65 | 52 | fail |
| Comparative spunbound/submicron meltblown/spunbound | unknown | 43 | 110 | fail |
| Comparative non-breathable polyethylene film | unknown | 0.0072 | > 1,000 | pass |
| Comparative ePTFE tentered special membrane | 8 | 3.18 | 693 | pass |
| Comparative ePTFE comparative ePTFE tentered special membrane | 15 | 1.67 | 805 | pass |
| First Exemplary Embodiment | 20 | 8.14 | 442 | pass |
| First Exemplary Embodiment | 40 | 6.24 | 474 | pass |
| First Exemplary Embodiment | 60 | 4.43 | 533 | pass |
| Fifth Exemplary Embodiment | 22 | 9.96 | 395 | pass |
| Fifth Exemplary Embodiment | 68 | 2.98 | 664 | pass |

Referring to Table 16, all samples of the exemplary embodiments exhibited a hydro head value of 395 cm or above, and passed the blood penetration test. In contrast, none of the submicron melt blown nonwovens that was tested exhibited hydro head above 110 cm, and they all failed the blood penetration test. All of the tested ePTFE membranes exhibited a low air permeability of 3.5 cfm or less. The samples of the exemplary embodiments having a thickness of about 20µ had a hydro head of about 395 cm to about 442 cm and passed the blood penetration test while providing air permeability in the range of about 8 cfm to about 10 cfm. These air permeabilities are much higher than for the ePTFE membranes, indicating that the espun PTFE nonwoven fabrics seek to provide better comfort by allowing water vapor to escape, such as during long surgical procedures. For example, the 20 µ thick sample of the first exemplary embodiment had a high hydro head value and successfully passed the blood penetration test while maintaining a high air permeability, which may be advantageous in medical protective fabrics.

As another specific example of how espun mats of this disclosure (e.g., see Figs. 2-6, 9 and 10) may be used, tissue scaffolding may be based solely on (e.g., may consist of, or consist essentially of) espun PTFE, or tissue scaffolding may comprise espun PTFE in combination with other suitable media. In accordance with one aspect of this disclosure, tissue scaffolds are materials with structural and functional properties that can be used to replace or correct damaged, missing or poorly functioning components in living systems. The tissue scaffolds may be configured for supporting (e.g., directing) the growth of cells implanted in or on the tissue scaffolds and/or for supporting (e.g., directing) the growth of cells migrating to the tissue scaffolds from surrounding tissue. For example, such cell growth may extend into the pores of the tissue scaffolding (e.g., espun mats) so that the porous structure provides a substrate for cell attachment and proliferation.

In accordance with one aspect of this disclosure, the three-dimensional and porous characteristics of the espun PTFE nonwoven fabric coupled with the biocompatibility of the espun PTFE nonwoven fabric allow the espun PTFE nonwoven fabric to function well as tissue scaffolding when the pore size of the espun PTFE nonwoven fabric is within a suitable range. For example, the pore structure of the espun PTFE nonwoven fabric may be configured to allow ready access for the entry of cells and nutrients that allow for additional tissue development and growth into and throughout the espun PTFE nonwoven fabric. In addition and/or alternatively, the tortuous pathway defined by the pores of the espun PTFE nonwoven fabric, density of the espun PTFE nonwoven fabric, and fiber diameter of the espun PTFE nonwoven fabric can be controlled to inhibit cell ingrowth, as (e.g., if) desired. In one example, the pore size(s) of the espun PTFE nonwoven fabric may be in a range from less than 0.2 microns to 10 microns. In this regard, pore size(s) less than 0.2 microns may inhibit cell growth into the pores of the espun PTFE nonwoven fabric. Accordingly, if restricting such cell ingrowth is desired, the pore size(s) of the espun PTFE nonwoven fabric may be less than about 0.2 microns; whereas if cell ingrowth is desired the pore size(s) of the espun PTFE nonwoven fabric may be in a range from about 0.2 microns to about 10 microns. Other pore sizes may be utilized in accordance with other aspects of this disclosure. The tissue scaffolding, or the like, of this disclosure may be used, for example, in or as grafts; stent-grafts; connective scaffolding for positioning or protecting organs, or bridging of torn tissues such as muscles or tendons; replacement of diseased or damaged tissue; implantable devices for drug delivery or cell therapy; wound care; regenerative membranes; and/or in any other suitable manner.

In some embodiments of this disclosure, the nonwoven fabrics shown in each of Figs. 2-6, 9 and 10 may be characterized as being tissue scaffolding, wherein living cells may grow on outer surfaces of the nonwoven fabrics and/or in pores of the nonwoven fabrics.

For each of the articles, constructs, or the like, discussed above, it may comprise, consist essentially of, or otherwise incorporate the nonwoven material of this disclosure, unless indicated otherwise. In this regard, for each of the articles, constructs, or the like, that may comprise, consist essentially of, or incorporate the nonwoven material of this disclosure, in some examples the article, construct, or the like, is conventional, except that one or more materials thereof are replaced with one or more of the nonwoven materials of this disclosure.

Reiterating from above, throughout this Detailed Description section and the figures of this disclosure, measurements or values that are not explicitly specified as being approximate (e.g., "about") may alternatively be approximate (e.g., "about").

Many modifications and other embodiments of the invention will come to mind to one skilled in the art to which this invention pertains having the benefit of the teachings presented in the foregoing description. Therefore, it is to be understood that the above examples are in no way intended to limit the scope of the present invention. It will be understood by those skilled in the art that while the present disclosure has been discussed above with reference to exemplary embodiments, various additions, modifications and changes can be made thereto without departing from the spirit and scope of the invention as set forth in the claims.

## Claims

1. A filter comprising:
a support structure; and
a mat comprising electrospun poly(tetrafluoroethylene), the mat being supported by the support structure.

2. A filtration medium comprising:
a mat comprising electrospun poly(tetrafluoroethylene), the mat being configured so that the mat is capable of meeting HEPA standard IEST-RP-CC001.3, the electrospun poly(tetrafluoroethylene) comprising nonwoven fibers having an average fiber diameter between about 250 nm and about 1500 nm, and an average thickness of the mat being about 200 µm or less.

3. The filtration medium of claim 2, wherein the mat comprises a nonwoven fabric, and the nonwoven fabric comprises the electrospun poly(tetrafluoroethylene).

4. The filtration medium of claim 2, wherein the average fiber diameter is about 500 nm or less or wherein the average fiber diameter is about 400 nm or less.

5. The filtration medium of claim 2, wherein the mat is a mat of the electrospun poly(tetrafluoroethylene), and an average thickness of the electrospun poly(tetrafluoroethylene) mat is about 100 µm or less.

6. The filtration medium of claim 5, wherein the electrospun poly(tetrafluoroethylene) mat is configured for providing a pressure drop of about 40 mm H₂O or less when tested under HEPA MIL-STD 282 using 0.3 µm particles, 5.3 cm/s velocity, and a 100 cm² area of the electrospun poly(tetrafluoroethylene) mat in a flat configuration, or wherein the electrospun poly(tetrafluoroethylene) mat is configured for providing a pressure drop of about 4 mm H₂O or less when tested under HEPA MIL-STD 282 using 0.3 µm particles, 5.3 cm/s velocity, and a 100 cm² area of the electrospun poly(tetrafluoroethylene) mat in a flat configuration.

7. The filtration medium of claim 5, wherein the density of the electrospun poly(tetrafluoroethylene) mat is about 30 grams per square meter per mil thickness or less, in particular about 25 grams per square meter per mil thickness or less, in particular about 20 grams per square meter per mil thickness or less, in particular about 10 grams per square meter per mil thickness or less.

8. A method for preparing a filtration medium, comprising:
having a dispersion comprising
a fluorinated polymer in particulate form with a given average particle size;
a fiberizing polymer;
a dispersion medium; and
an optional conductive species; such that the dispersion has a given conductivity; and
electrospinning said dispersion to provide a polymeric mat capable of meeting HEPA standard IEST-RP-CC001.3.

9. The method of claim 8, wherein the fluorinated polymer is poly(tetrafluoroethylene) or wherein the fluorinated polymer is selected from the group consisting of fluorinated ethylene propylene, polyvinylidene fluoride, perfluoroalkoxy, a copolymer of tetrafluoroethylene, hexafluoropropylene and vinylidene fluoride (THV), poly(ethylene-co-tetrafluoroethylene), ethylene chlorotrifluoroethylene, polychlorotrifluoroethylene, and copolymers, blends, and derivatives thereof.

10. The method of claim 8, wherein the given average particle size of the fluorinated polymer is about 230 nm or less, in particular about 160 nm or less, in particular about 130 nm or less, in particular about 80 nm or less.

11. The method of claim 8, wherein the electrospinning step is performed for a period of time such that the polymeric mat has an average thickness of about 200 µm or less, in particular of about 100 µm or less.

12. A filtration medium comprising:
a nonwoven, electrospun poly(tetrafluoroethylene) mat capable of functioning as an ULPA filter so as to remove from air at least 99.999% of airborne particles with particle sizes of less than 0.3 µ, wherein
the electrospun poly(tetrafluoroethylene) mat comprises nonwoven fibers having an average fiber diameter between about 250 nm and about 1500 nm, and
an average thickness of the electrospun poly(tetrafluoroethylene) mat is about 200 µm or less.

13. A method for preparing a filtration medium, comprising:
having a dispersion comprising:
a fluorinated polymer in particulate form with a given average particle size;
a fiberizing polymer;
a dispersion medium; and
an optional conductive species; such that the dispersion has a given conductivity; and
electrospinning said dispersion to provide a polymeric mat capable of functioning as an ULPA filter so as to remove from air at least 99.999% of airborne particles with a particle size of less than 0.3 microns.

14. A mat comprising electrospun poly(tetrafluoroethylene), the mat comprising:
an average fiber diameter of less than about 1003 nm; and
a density of greater than about 8.1 grams per square meter per mil thickness or less.

15. A separation membrane or fabric or tissue scaffold comprising the mat of claim 14.

16. A method for preparing a mat, comprising:
having a dispersion comprising
a fluorinated polymer in particulate form with an average particle size of less than about 230 nm,
a fiberizing polymer,
a dispersion medium, and
a conductivity of more than about 250 µS/cm;
electrospinning the dispersion to provide the mat.

17. A gradient fabric comprising:
two or more layers of electrospun poly(tetrafluoroethylene) fibers, wherein the two or more layers comprise at least two layers having different densities, such that a cross-section of the fabric exhibits one or more density gradients.

18. A nonwoven fabric comprising:
electrospun poly(tetrafluoroethylene), the nonwoven fabric being configured so that the nonwoven fabric is capable of both
passing the blood penetration test of ASTM F1670, and
providing air permeability of at least about 2.5 cfm.
